(19) Europäisches Patentamt
European Patent Office
Office européen des brevets

(11) EP 2 408 849 B1

(12) **EUROPÄISCHE PATENTSCHRIFT**

(45) Veröffentlichungstag und Bekanntmachung des Hinweises auf die Patenterteilung:
**03.06.2015 Patentblatt 2015/23**

(21) Anmeldenummer: **10717471.6**

(22) Anmeldetag: **17.03.2010**

(51) Int Cl.:
***C08J 3/24*** *(2006.01)* ***C08J 3/26*** *(2006.01)*
***C08J 5/02*** *(2006.01)* ***A61B 19/04*** *(2006.01)*
***A61F 6/04*** *(2006.01)* ***B01J 19/12*** *(2006.01)*
***B01J 19/24*** *(2006.01)* ***C08J 3/28*** *(2006.01)*
***B29C 41/14*** *(2006.01)*

(86) Internationale Anmeldenummer:
**PCT/AT2010/000081**

(87) Internationale Veröffentlichungsnummer:
**WO 2010/105283 (23.09.2010 Gazette 2010/38)**

(54) **VERFAHREN ZUR HERSTELLUNG EINES VERNETZTEN ELASTOMERS**

PROCESS FOR MAKING A CROSSLINKED ELASTOMER

PROCÉDÉ DE PRÉPARATION D'UN ÉLASTOMÈRE RÉTICULÉ

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.03.2009 AT 4212009**

(43) Veröffentlichungstag der Anmeldung:
**25.01.2012 Patentblatt 2012/04**

(73) Patentinhaber: **Semperit AG Holding**
**1031 Wien (AT)**

(72) Erfinder:
- **HOLZNER, Armin**
  **A-2630 Ternitz (AT)**
- **KERN, Wolfgang**
  **A-8055 Seiersberg (AT)**
- **SCHALLER, Raimund**
  **A-2620 Neunkirchen (AT)**
- **SCHLÖGL, Sandra**
  **8152 Stallhofen (AT)**

(74) Vertreter: **Burger, Hannes**
**Anwälte Burger & Partner**
**Rechtsanwalt GmbH**
**Rosenauerweg 16**
**4580 Windischgarsten (AT)**

(56) Entgegenhaltungen:
**EP-A2- 1 762 586** **DE-A1-102005 043 222**
**GB-A- 2 239 247** **US-A1- 2003 141 457**
**US-B1- 6 329 444**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 408 849 B1

**Beschreibung**

Die Erfindung betrifft ein Verfahren zur Herstellung eines vernetzten Elastomers durch Bestrahlung einer Polymerdispersion aus zumindest einem vernetzbaren Polymer mit elektromagnetischer Strahlung im ultravioletten (UV-Licht) und/oder sichtbaren Spektralbereich, wobei die Vernetzung in zumindest zwei Schritten als Vorvernetzung und Nachvernetzung durchgeführt wird und der Polymerdispersion zumindest ein Fotoinitiator zur Auslösung der Vernetzungsreaktion vor der Vorvernetzung zugesetzt wird, ein Verfahren zur Herstellung eines Tauchartikels aus zumindest einem Latex, insbesondere eines Handschuhs oder eines Kondoms, bei dem eine Form mit einer äußeren Kontur, die dem herzustellenden Tauchartikel entspricht, für eine vorbestimmbare Zeit in ein Tauchbad, enthaltend den zumindest einen Latex, getaucht wird und danach der Tauchartikel verfestigt und/oder getrocknet wird, eine Vorrichtung zur Herstellung eines Tauchartikels aus einem Latex, umfassend einen Reaktor, zumindest eine Tauchform und zumindest ein Tauchbad, wobei dem Reaktor zumindest eine erste Strahlungsquelle zur Abgabe von elektromagnetischer Strahlung im ultravioletten (UV-Licht) und/oder sichtbaren Spektralbereich zugeordnet ist, sowie einen Handschuh aus einem vernetzten Elastomer.

Um Elastomeren die spezifischen elastischen Eigenschaften zu verleihen, müssen die Polymerketten des Elastomers zumindest partiell miteinander vernetzt werden. Üblicherweise erfolgt die Vernetzung über Doppelbindungen, die sich entweder in der Hauptkette, wie beispielsweise bei Polyisopren, Polybutadien, Styrol-Butadien-Kautschuk, Chloropren, Nitril-Butadien-Kautschuk, oder einer Seitenkette, wie z.B. bei EPDM, des Elastomers befinden. Großtechnisch wird die Vernetzung von Elastomeren, darunter auch Kautschuk-Latex, bislang auf drei verschiedene Arten durchgeführt, nämlich durch die Schwefelvernetzung, die peroxidische Vernetzung oder mit Strahlungsvernetzungsverfahren.

Aus der von der Anmelderin stammenden EP 1 762 586 A2 ist weiters ein Verfahren zur Herstellung von Handschuhen durch UV-Vernetzung eines Kautschuks bekannt. Dazu wird der Latex in einem Fallfilmreaktor der UV-Strahlung ausgesetzt und danach werden die Handschuhe in üblicher Art und Weise getaucht. Es ist darin auch die Möglichkeit der Nachvernetzung angesprochen, allerdings nicht weiter ausgeführt. Von Vorteil ist dabei, dass keine üblichen Prozesschemikalien verwendet werden müssen, wie z.B. die angesprochenen Organoschwefelverbindungen, wodurch das Potential für Kontaktallergien derartiger Handschuhe gesenkt werden kann.

Aufgabe vorliegender Erfindung ist es, dieses UV-Vernetzungsverfahren weiter zu verbessern.

Diese Aufgabe der Erfindung wird - jeweils eigenständig - dadurch gelöst, dass bei dem erfindungsgemäßen Verfahren der vorvernetzten Polymerdispersion vor und/oder während der Nachvernetzung nochmals zumindest ein Fotoinitiator zugesetzt und die Nachvernetzung ebenfalls mit elektromagnetischer Strahlung im ultravioletten (UV-Licht) und/oder sichtbaren Spektralbereich durchgeführt wird, dass weiters bei dem Verfahren zur Herstellung eines Tauchartikels der Latex nach diesem Verfahren vernetzt wird, dass bei der Vorrichtung nach dem zumindest einem Tauchbad eine weitere Strahlungsquelle zur Abgabe von elektromagnetischer Strahlung im ultravioletten (UV-Licht) und/oder sichtbaren Spektralbereich angeordnet ist, sowie durch den Handschuh der nach diesem Verfahren hergestellt ist und eine Reißfestigkeit von zumindest 14 $N/mm^2$, insbesondere zumindest 20 $N/mm^2$, vorzugsweise zumindest 25 $N/mm^2$, aufweist.

Überraschenderweise wurde gefunden, dass durch die Aufteilung der UV-Vernetzung auf zwei Einzelvernetzungsschritte, nämlich die Vorvernetzung und die Nachvernetzung, wobei beide Teilvernetzungen mittels Strahlung im UV- und/oder VIS-Bereich erfolgen, die mechanische Festigkeit der Elastomerfilme, insbesondere die Reißfestigkeit, gegenüber der einmaligen Bestrahlung mit UV-Licht verbessert werden kann. Zudem ist durch diese Aufteilung eine verbesserte Prozessführung zur Herstellung von Handschuhen erzielbar, indem die Filmbildung in der Vorvernetzungsstufe verbessert werden kann. Nach wie vor weisen die damit hergestellten Produkte, also beispielsweise Untersuchungshandschuhe, Operationshandschuhe, Kondome, Katheter, Infusionsschläuche, Anästhesiemasken, etc., ein geringes bzw. kein Typ IV Allergiepotential auf. Zudem kann die Alterungsbeständigkeit des Elastomers verbessert werden. Auch hinsichtlich hochenergetischer Strahlung weisen die Elastomerprodukte eine verbesserte Stabilität auf. Dies ist insbesondere in Hinblick auf die Sterilisation der Medizinprodukte mit Gammastrahlung von Bedeutung. Zudem konnte durch diese Zweiteilung der Vernetzung der Gesamtenergieverbrauch, der allein aufgrund der Tatsache, dass die UV-Vernetzung bei Raumtemperatur stattfinden kann, schon entsprechend geringer ist, als bei alternativen Vernetzungsverfahren, deutlich reduziert werden. Darüber hinaus können durch die Nachvernetzung eventuell vorhandene Konzentrationen an Restchemikalien durch kovalente Bindung der Restchemikalien an den Latex verringert werden. Durch die höheren Reißfestigkeiten der Filme können diese mit einer geringeren Wandstärke hergestellt werden, wodurch die Wirtschaftlichkeit des Verfahrens verbessert werden kann.

Gemäß einer Ausführungsvariante ist vorgesehen, dass die zugesetzte Menge des zumindest einen Fotoinitiators in der Vorvernetzung maximal gleich groß ist, bevorzugt kleiner ist, als die Menge des zumindest einen Fotoinitiators, die für die Nachvernetzung verwendet wird. Es kann damit die Filmbildung, z.B. auf der Handschuhform beim Tauchverfahren, verbessert werden, da in der Vorvernetzung ein Zwischenprodukt erzeugt wird, dessen mechanische Festigkeit noch deutlich geringer ist, als jene des Endproduktes, insbesondere aufgrund der geringeren Anzahl an Vernetzungsstellen, und somit das Fließverhalten des Latex bzw. Gels sich positiv auf die, insbesondere gleichmäßige,

Filmbildung auswirkt.

Es sei jedoch darauf hingewiesen, dass die zugesetzte Menge des Fotoinitiators bzw. der Fotoinitiatoren in der Vorvernetzung größer ist als in der Nachvernetzung, wenngleich die voranstehende Ausführungsvariante bevorzugt wird.

Insbesondere hat sich dabei für die Herstellung von medizinischen Latexprodukten, insbesondere Handschuhen, als vorteilhaft herausgestellt, wenn für die Vernetzungsreaktion des Latex bzw. der Latexmischung im Hinblick auf ausgewogene mechanische Eigenschaften, d.h. insbesondere eine gewünschte Festigkeit bei entsprechender Relaxation des Handschuhs, d.h. Abbau des Druckes bzw. der Kraft auf die Hand nach der Dehnung infolge des Anziehens des Handschuhs und damit Vermeidung eines "Einschnürgefühls" beim Träger bzw. um die entsprechende Taktilität für den Handschuhträger zu gewährleisten, wenn der Anteil des zumindest einen Fotoinitiators an der Polymerdispersion für die Vorvernetzung zwischen 0,2 phr und 5,0 phr beträgt bzw. wenn gemäß einer weiteren Ausführungsvariante der Anteil des zumindest einen Fotoinitiators an der Polymerdispersion für die Nachvernetzung zwischen 0,5 phr und 5,0 phr beträgt. Unterhalb der unteren Grenzwerte ist die Vernetzung für die gewünschten mechanischen Eigenschaften zu gering. Konzentrationen von mehr als 5,0 phr für die Vor- bzw. Nachvernetzung gehen einerseits zu Lasten der Wirtschaftlichkeit des Verfahrens und andererseits wird die Vernetzungsstellendichte zu groß.

Zur Verbesserung der Reißfestigkeit und auch aus Wirtschaftlichkeitsgründen ist es auch von Vorteil wenn der Anteil des zumindest einen Fotoinitiators an der Polymerdispersion für die Vorvernetzung zwischen 0,5 phr und 3,5 phr, insbesondere zwischen 0,75 phr und 1,2 phr, beträgt bzw. wenn gemäß einer weiteren Ausführungsvariante der Anteil des zumindest einen Fotoinitiators an der Polymerdispersion für die Nachvernetzung zwischen 0,8 phr und 3,75 phr, insbesondere zwischen 1 phr und 1,5 phr, beträgt.

Vorzugsweise wird die Polymerdispersion in der Vorvernetzung zumindest zweimal bestrahlt. Überraschenderweise weist das fertige Elastomer nach zwei- oder dreimaliger Bestrahlung homogenere Eigenschaften, wie z.B. die mechanische Reißfestigkeit, auf. Dazu kann die erfindungsgemäße Vorrichtung zwei oder drei Reaktoren in Fließrichtung des Latex hintereinander geschaltet aufweisen, um eine kontinuierliche Produktion zu ermöglichen. Bei Versuchen mit vier oder mehr Belichtungszyklen wurde gefunden, dass sich die Reißfestigkeit des fertigen Produktes nicht in dem Ausmaß verändert, wie sich die Wirtschaftlichkeit des Verfahrens verringert. Zudem kann gegebenenfalls auch eine Übervernetzung in der Vorvernetzungsphase beobachtet werden. Bei einer einmaligen Bestrahlung kann die Reißfestigkeit durch eine geringere Vorvernetzung ebenfalls geringer sein.

Bevorzugt wird ein Fotoinitiator eingesetzt, der aus einer Gruppe umfassend 2-Hydroxy-2-methyl-1-phenylpropanon (Handelsname: Genocure DMHA; Rahn AG), Phenylglyoxalsäuremethylester, 2,4,6-Trimethylbenzoylphenylphosphinsäure Ethylester (Handelsname: Lucirin TPO L; BASF), Methylbenzoylformiat (Handelsname: Genocure MBF; Rahn AG), 1-[4-(2-Hydroxyethoxy)-phenyl]-2-hydroxy-methyl-1-propanon-1-on (Handelsname: Irgacure 2959; Rahn AG) 2-Dimethylamino-2-(4-methylbenzyl)-1-(4-morpholin-4-ylphenyl)-butan-1-on (Handelsname: Irgacure 379; CIBA), 2 Methyl-1-[4-(methylthio)phenyl]-2-morpholinopropan-1-on (Handelsname: Irgacure 907; CIBA), 2,4,6-Trimethylbenzoyldiphenylphosphinoxid (Handelsname: Lucirin TPO; BASF), ausgewählt ist. Von Vorteil ist dabei einerseits die gute Hautverträglichkeit und die Unbedenklichkeit in Hinblick auf die Lebensmittelechtheit, sodass für den Fall, dass geringe Mengen im fertigen Elastomer verbleiben, keine Probleme entstehen, wodurch die Produktion, d.h. die Waschschritte vereinfacht werden können. Daneben haben diese Fotoinitiatoren eine hohe Reaktivität, sodass eine effiziente Vernetzung bei geringer Strahlerleistung bzw. bei niedriger Fotoinitiatorkonzentration durchgeführt werden kann, wodurch ebenfalls die Prozesskosten verringert werden können. Außerdem weisen diese Fotoinitiatoren eine hohe thermische Stabilität auf. Lucirin TPO (L) und Genocure DMHA bieten zudem den Vorteil, dass diese in flüssiger Form erhältlich sind und damit entsprechend einfacher mit weiteren Prozesschemikalien, wie z.B. Stabilisatoren, in wässrigen Systemen emulgiert werden können. Zudem erstreckt sich der Absorptionsbereich von Lucirin TPO L bis über 400 nm, sodass längerwelliges Licht für die Bestrahlung verwendet werden kann. Dies wiederum hat den Vorteil, dass die Strahlung auch in tiefere Schichten vordringen kann. Es können damit dickwandigere bzw. stark pigmentierte Elastomerfilme bzw. -produkte hergestellt werden.

Nach einer Ausführungsvariante der Erfindung wird die Vorvernetzung an einem Film durchgeführt, insbesondere wenn Naturkautschuk vernetzt wird. Bevorzugt beträgt die Schichtdicke zwischen 0,1 mm und 2 mm. Es sind damit auch Latexarten kontinuierlich verarbeitbar, die eine hohe bzw. höhere Absorption aufweisen. Durch die geringe Schichtdicke während der Vorvernetzung kann die zu bestrahlende Schicht zumindest annähernd homogen durchstrahlt werden.

Insbesondere beträgt die Schichtdicke des Filmes während der Vorvernetzung zwischen 0,3 mm und 0,6 mm.

Durch die Verwendung von zumindest zwei Strahlungsquellen in der Nachvernetzung können homogenere Dosisverteilungen bei dreidimensional geformten Produkten, wie z.B. Handschuhen, auch im großtechnischen Maßstab erreicht werden. Beispielsweise werden zwischen drei und acht, insbesondere sechs Strahler, in Produktionsrichtung hintereinander in der Vorrichtung angeordnet. Selbstverständlich können aber auch mehr als acht Strahler angeordnet werden.

Es ist weiters von Vorteil, wenn die Nachvernetzung mit einer höheren Strahlungsdosis durchgeführt wird, als die Vorvernetzung. Damit können in der Vorvernetzung geringere Vernetzungsgrade ermöglicht werden, wodurch die

Filmbildung, z.B. an einer Tauchform, verbessert werden kann, indem die Latexpartikel besser ineinander fließen und miteinander verschmelzen können, und werden die höheren Reißfestigkeiten in der Folge durch die höhere Strahlungsdosis in der Nachvernetzung erreicht.

Dabei ist insbesondere von Vorteil, beispielsweise bei der Vernetzung von IR-Latex, wenn die Nachvernetzung mit einer Strahlungsdosis durchgeführt wird, die zwischen 150 % und 500 % der Strahlungsdosis der Vorvernetzung beträgt.

Bevorzugt wird die Nachvernetzung mit einer Strahlungsdosis durchgeführt, die zwischen 200 % und 300 % der Strahlungsdosis der Vorvernetzung beträgt.

Für die Vorvernetzung und/oder die Nachvernetzung wird dem Latex bzw. dem vorvernetzten Latex zumindest ein Covernetzer mit zumindest einer Thiolgruppe zugesetzt. Es ist damit der Ablauf der Vernetzung über eine (radikalische) Thiol-en Reaktion möglich, wodurch die Sauerstoffinhibierung der Vernetzung verringert werden kann, sodass eine Vernetzung an Luft erfolgen kann. Zudem setzt der Gelpunkt verzögert ein, wodurch eine höhere Vernetzung und damit eine höhere mechanische Reißfestigkeit erreicht werden können.

Aus den voranstehend genannten Gründen zu den Mengenverhältnissen des Fotoinitiators in der Vorvernetzung und der Nachvernetzung ist es von Vorteil, wenn die zugesetzte Menge des zumindest einen Covernetzers in der Vorvernetzung maximal gleich groß, bevorzugt kleiner, ist, als die Menge des zumindest einen Covernetzers, die für die Nachvernetzung verwendet wird, wobei der Anteil des zumindest einen Covernetzers an der Polymerdispersion für die Vorvernetzung vorzugsweise zwischen 0,5 phr und 2,0 phr beträgt bzw. der Anteil des zumindest einen Covernetzers an der Polymerdispersion für die Nachvernetzung zwischen 0,5 phr und 2,5 phr beträgt.

Zur Verbesserung der Reißfestigkeit und auch aus Wirtschaftlichkeitsgründen ist es auch von Vorteil wenn der Anteil des zumindest einen Covernetzers an der Polymerdispersion für die Vorvernetzung zwischen 0,1 phr und 1,5 phr bzw. zwischen 0,2 phr und 1,2 phr, beträgt bzw. wenn gemäß einer weiteren Ausführungsvariante der Anteil des zumindest einen Covernetzers an der Polymerdispersion für die Nachvernetzung zwischen 0,9 phr und 2 phr bzw. zwischen 1,2 phr und 1,5 phr beträgt.

Bevorzugt wird als Covernetzer Trimethylolpropan-tris-3-mercaptopropionat oder Pentaerythritol tetrakis-3-mercaptopropionat verwendet, da diese eine relativ hohe Reaktivität aufweisen, wobei Trimethylolpropan-tris-3-mercaptopropionat eine höhere Reaktivität als Pentaerythritol tetrakis-3-mercaptopropionat aufweist, Pentaerythritol tetrakis-3-mercaptopropionat allerdings den Vorteil hat, dass es im Kontakt mit Lebensmitteln zugelassen ist. Zudem hat Pentaerythritol tetrakis-3-mercaptopropionat den Vorteil, dass die Gefahr einer Übervernetzung geringer ist, sodass damit auch ein dritter Belichtungszyklus in der Vorvernetzung möglich ist. Bei Anwendung von zwei Belichtungszyklen hat jedoch die Verwendung von Trimethylolpropan-tris-3-mercaptopropionat Vorteile gegenüber der Verwendung von Pentaerythritol tetrakis-3-mercaptopropionat, wobei prinzipiell der Einsatz beider Covernetzer, auch gleichzeitig, möglich ist.

Überraschenderweise kann der vorvernetzten Polymerdispersion vor der Nachvernetzung zumindest ein Alterungsschutzmittel zugesetzt werden, wodurch nicht nur die Alterungsbeständigkeit an sich verbessert werden kann, sondern es damit auch möglich ist, die Beständigkeit des Elastomers gegenüber hochenergetischer Strahlung, wie z.B. für die Sterilisation, zu verbessern.

Als Alterungsschutzmittel können insbesondere Vitamin E und/oder sterisch gehinderte phenolische Verbindungen, wie z.B. butylierte Reaktionsprodukte aus p-Kresol und Dicyclopentadien (RALOX ® LC, Fa. Solvadis), verwendet werden. Vitamin E ($\alpha$-Tocopherol) wirkt bekanntlich als Radikalfänger. Zudem bietet es den Vorteil, dass es in Hinblick auf die humane Verträglichkeit unbedenklich ist, damit vielmehr auch ein Zusatznutzen für den Anwender der fertigen Elastomerartikel, beispielsweise Handschuhe, realisiert werden kann. Vitamin E hat aber auch den Vorteil, dass es die Thiol-en Reaktion nur geringfügig inhibiert.

Zur Erhöhung der Effizienz der Nachvernetzung ist es von Vorteil, wenn zwischen der Vorvernetzung und der Nachvernetzung eine Trocknung des vorvernetzten Elastomers bis zu einer Restfeuchte von maximal 6 % durchgeführt wird. Es wird damit die Lichtstreuung des Latexfilmes reduziert, sodass das Anregungslicht in tiefere Schichten eindringen kann.

Zur weiteren Verbesserung dieses Effektes und damit einer weiteren Erhöhung der Reißfestigkeit ist es von Vorteil, wenn die Trocknung bis zu einer Restfeuchte von maximal 4 %, bezogen auf den Film, durchgeführt wird.

Der Polymerdispersion kann vor der Vorvernetzung zumindest ein Tensid mit zumindest einem photochemisch aktiven Zentrum, insbesondere mit einer Doppelbindung, z.B. Linolsäure, zugesetzt werden, um die Filmbildung des Latex zu verbessern.

Für die Vernetzung, insbesondere die Nachvernetzung, kann für die Bestrahlung eine mit Gallium dotierte Quecksilber-Hochdruckdampflampe verwendet werden. Durch die Galliumdotierung wird eine Verschiebung des Emissionsbereiches der Lampe zu längeren Wellenlängen erreicht, der sich bis in den sichtbaren Bereich erstrecken kann. Damit können auch tiefer liegende Schichten des Latex besser vernetzt werden, insbesondere bei Verwendung von Lucirin TPO L. Es hat sich damit auch gezeigt, dass die Oberfläche des Latexfilmes weniger durch einen Ozonangriff geschädigt wird, sodass die Alterungsbeständigkeit weiter verbessert werden kann.

Es ist weiters möglich, dass die Nachvernetzung unter Inertgasatmosphäre durchgeführt wird, beispielsweise

Argon oder Stickstoff, um eine Verschlechterung der Oberflächeneigenschaften durch den Einfluss von Sauerstoff zu vermeiden.

Die Formgebung des Latex durch Tauchen der Form in das Tauchbad kann bei dem Verfahren zur Herstellung eines Tauchartikels zwischen der Vorvernetzung und der Nachvernetzung des Latex durchgeführt werden, wodurch bessere Filmqualitäten erreicht werden.

Dabei ist es von Vorteil, wenn die Nachvernetzung auf der Form durchgeführt wird, d.h. dass sich der Film während der Nachvernetzung auf der Form befindet. Obwohl die homogene Bestrahlung in diesem Fall erschwert ist, hat diese Verfahrensweise jedoch den Vorteil, dass die Filme nicht von den Formen abgezogen werden müssen, sodass einerseits die Wirtschaftlichkeit des Verfahrens verbessert werden kann, und andererseits Probleme bei der Handhabung von klebrigen Filmen vermieden werden können. Dabei hat es sich als vorteilhaft erwiesen, wenn die Form während der Nachvernetzung in unterschiedlichen Winkeln zur Strahlungsquelle ausgerichtet wird. Es kann damit die Homogenität der Bestrahlung verbessert werden, insbesondere Schatteneffekte aufgrund der Dreidimensionalität des Produktes besser vermieden werden.

Gemäß einer Ausführungsvariante der Vorrichtung ist vorgesehen, dass nach dem zumindest einen Tauchbad eine Rolliereinheit angeordnet ist, mit der die Tauchartikel zumindest teilweise, insbesondere bei Handschuhen das offene Ende des Schaftes, gerollt werden können, und dass die weitere Strahlungsquelle der Rolliereinheit zugeordnet ist. Es wird damit erreicht, dass die Tauchform und der darauf befindliche Elastomerfilm nicht nur während der translatorischen Vorwärtsbewegung bestrahlt wird, sondern dass der translatorischen Bewegung eine Rotationsbewegung überlagert wird, wodurch die Bestrahlung des Elastomerfilmes, insbesondere die Dosisverteilung über die Handschuhoberfläche homogener erfolgen kann. Die Geschwindigkeit der translatorischen Vorschubbewegung kann dabei zwischen 1m/min und 25 m/min betragen, jene der Rotationsbewegung zwischen 25 U/m Vorschub und 50 U/m Vorschub.

Aus voranstehend genannten Gründen werden für die Vorvernetzung vorzugsweise zumindest zwei Reaktoren mit jeweils zumindest einer ersten Strahlungsquelle in Produktionsrichtung hintereinander in der Vorrichtung angeordnet.

Zwischen den beiden Reaktoren der Vorrichtung kann zumindest ein Behälter angeordnet sein, der gegebenenfalls ein Rührwerk aufweist. Es ist damit auf einfache Weise möglich Prozesschemikalien zwischen den beiden Belichtungszyklen nachzudosieren bzw. zuzudosieren.

Es sei an dieser Stelle angemerkt, dass auch weitere Reaktoren verwendet werden können, z.B. ein dritter Reaktor um bei einer kontinuierlichen Reaktionsführung Reinigungsarbeiten an einem der beiden anderen Reaktoren durchführen zu können.

Es ist möglich einen Spektralbereich für die Vernetzung zu verwenden, mit Wellenlängen ausgewählt aus einem Bereich mit einer unteren Grenze von 150 nm, insbesondere 250 nm, vorzugsweise 275 nm, und einer oberen Grenze von 600 nm, insbesondere 475 nm, vorzugsweise 400 nm.

Neben den voranstehend genannten Fotoinitiatoren können prinzipiell aber auch andere Fotoinitiatoren eingesetzt werden, die im ultravioletten und/oder sichtbaren Spektralbereich, insbesondere im an den UV-Bereich anschließenden Blaubereich des sichtbaren Spektralbereichs, eine entsprechende Reaktion zeigen. Beispiele hierfür sind in der eingangs genannten EP 1 762 586 A2 enthalten.

Neben den voranstehend genannten, bevorzugt genannten Covernetzern können auch andere Covernetzer eingesetzt werden, um die Vernetzung besser gestalten zu können. Beispielsweise kann als Covernetzer auch zumindest ein Selenol, wie z.B. 1,6 Hexandiselenol, oder andere Thiole, insbesondere jeweils mehrfach funktionelle Verbindungen bzw. Derivate davon, wie z.B. Bisthiole, beispielsweise 1,6-Hexandithiol, Tristhiole, Bisselenole, Trisselenole, sowie Mischungen daraus verwendet werden.

In dem erfindungsgemäßen Verfahren kann zumindest ein weiteres Hilfsmittel verwendet werden, ausgewählt aus einer Gruppe umfassend, insbesondere mehrfach funktionelle, Acrylate, wie z.B. Hexandioldiacrylat (HDDA), Trimethylolpropantriacrylat (TMPTA), Verbindungen mit Vinyl- oder Allylgruppen, wie z.B. Triallyl-Cyanurat, Triallyl-Isocyanurat, sowie Mischungen daraus, wie z.B. zumindest ein genanntes, insbesondere mehrfach funktionelles, Acrylat mit zumindest einer genannten Verbindung mit Vinyl- oder Allylgruppen, um damit das Vernetzungsverhalten zu verbessern, wobei wiederum mehrfach funktionelle Verbindungen bevorzugt werden. Derartige Vernetzungs(hilfs)mittel können in einem Anteil bis zu 10 phr in der Polymerdispersion für die Vorvernetzung und/oder in der Polymerdispersion für die Nachvernetzung enthalten sein.

Es ist auch möglich zumindest einen Sensibilisator zuzusetzen, um die Lichtenergie besser auf den Fotoinitiator übertragen zu können und damit die Vernetzungsreaktion insgesamt zu beschleunigen bzw. den Ablauf positiv zu beeinflussen bzw. um damit auch die Möglichkeit zu schaffen, Fotoinitiatoren zu verwenden die in einem anderen Absorptionsbereich absorbieren als dieser für die gewünschte Reaktion vorteilhaft wäre.

Der zumindest eine Sensibilisator kann ausgewählt sein aus einer Gruppe umfassend organische Farbstoffe wie Eosin, aromatische Ketone, wie zum Beispiel Benzophenon oder Thioxanthon, kondensierte aromatische Verbindungen, wie zum Beispiel Anthracen oder Chrysen, anorganische Pigmente wie Zink-Phthalocyanin oder Titanoxide, sowie Mischungen daraus, da diese Verbindungen, insbesondere mit dem eingesetzten Fotoinitiator(en), eine entsprechende Wechselwirkung zeigen.

Der Anteil des Sensibilisators liegt bevorzugt in einem Bereich mit einer unteren Grenze von 0,1 % und einer oberen Grenze von 50 % des Anteils des Fotoinitiators, bzw. einem Bereich mit einer unteren Grenze von 10 % und einer oberen Grenze von 40 % des Anteils des Fotoinitiators oder einem Bereich mit einer unteren Grenze von 15 % und einer oberen Grenze von 25 % des Anteils.

Der zumindest eine Fotoinitiator und/oder der zumindest eine Covernetzer und/oder der zumindest eine Sensibilisator kann bzw. können vor der Zugabe zu dem zumindest einen Latex zu einer Voremulsion oder Vordispersion voremulgiert oder vordispergiert werden und kann zu diesem Zweck, um das Dispergier- bzw. Emulgierverhalten dieser Komponenten zu verbessern, zumindest ein Emulgator bzw. zumindest ein Dispergierhilfsmittel zugesetzt werden, wobei als Emulgator bzw. Dispergierhilfsmittel besonders bevorzugt ein Tensid verwendet wird, insbesondere Polyethylenglykol-sorbitan-monolaurat. Es wird damit der Eintrag dieser Komponenten in die, insbesondere flüssige, Latexphase erleichtert bzw. kann damit auch eine "Gleichverteilung" dieser Komponenten in der gesamten flüssigen Phase erreicht werden, wodurch höhere Reaktionsgeschwindigkeiten, d.h. höhere Umsätze pro Zeiteinheit, und damit eine Verkürzung des Verfahrens, erreicht werden kann.

Diese Voremulsion bzw. Vordispersion kann dem Latex bzw. Latexgemisch zumindest teilweise vor der Vernetzungsreaktion zugesetzt werden. Ebenso ist es möglich diese zumindest teilweise während der Vernetzungsreaktion bzw. zwischen den Belichtungszyklen der Vorvernetzung zu zudosieren. Es kann damit auf das Vernetzungsverhalten, insbesondere den Start der Vernetzungsreaktion, unterschiedlicher Latextypen entsprechend reagiert werden bzw. kann der Verbrauch an Hilfskomponenten bzw. an Starterkomponenten an den jeweils gewünschten Vernetzungsgrad besser angepasst werden. Insbesondere ist damit auch der Vernetzungsgrad des Latex bzw. des Latexgemisches besser einstellbar.

Der zumindest eine erfindungsgemäß zu vernetzende Latex kann ausgewählt sein aus einer Gruppe umfassend Naturkautschuk (NR), Polyisopren-Latex (IR), Nitrilbutadienkautschuk-Latex (NBR), Chloropren-Latex (CR), Styrol-Butadien Latex (SBR), Latices aus Ethylacrylat-Copolymeren (ACM), Latices aus Elastomeren welche durch Re-Emulgieren hergestellt werden, Latices aus funktionellen Copolymeren, wie z.B. fotoinitiatorhältige und/oder carboxylierte, Latices hergestellt aus Polymer-Blends, sowie Mischungen daraus, wobei mit diesen Latices - obwohl die Anwendung des erfindungsgemäßen Verfahrens auf andere Latextypen mit ungesättigten C-C Bindungen nicht ausgeschlossen sein soll - überraschender Weise entsprechend gute mechanische Eigenschaften bzw. entsprechende gute Eigenschaften erreicht werden konnten.

Dabei ist es von Vorteil, wenn ein Latex mit einem Feststoffgehalt vernetzt wird, der ausgewählt ist aus einem Bereich mit einer unteren Grenze von 20 % und einer oberen Grenze von 60 %, insbesondere wenn ein Latex mit einem Feststoffgehalt vernetzt wird, der ausgewählt ist aus einem Bereich mit einer unteren Grenze von 30 % und einer oberen Grenze von 50 %, vorzugsweise wenn ein Latex mit einem Feststoffgehalt vernetzt wird, der ausgewählt ist aus einem Bereich mit einer unteren Grenze von 35 % und einer oberen Grenze von 45 %, da mit diesem Feststoffgehalt(en) eine entsprechend gute Durchmischung der einzelnen Edukte und somit ein rascher Reaktionsablauf möglich ist. Zudem kann damit vermieden werden, dass die Schichtdicke im Reaktor der Vorvernetzung, insbesondere wenn ein Fallfilmreaktor verwendet wird, über die Bestrahlungslänge variiert. Oberhalb eines Feststoffgehaltes des Latex von 60 % wird der Latex zu viskos und neigt bereits zum Koagulieren. Unterhalb von 20 % ist der Feststoffgehalt zur Ausbildung einer gewünschten Schichtdicke beim Koagulations-Tauchverfahren zu gering.

Vorzugsweise wird die Vernetzungsreaktion in einem Fallfilmreaktor oder in einem Tauchreaktor durchgeführt, um bei vorbestimmbaren Schichtdicken des Reaktionsgemisches den Energieeintrag möglichst bis in die Kernbereiche der Mischung zu ermöglichen.

Als Energiequelle für elektromagnetische Strahlung können neben der voranstehend genannten bevorzugten dotierten Quecksilberdampflampe auch andere Quecksilberlampen, (gepulste) Xenonlampen, eine Excimer-Lampe, ein Laser, wie z.B. ein Excimer-Laser bzw. für die zumindest anteilsweise Vernetzungen im sichtbaren Blaubereich ein Laser, eine LED-Lichtquelle, verwendet werden. Ebenso können mikrowellenangeregte UV-Strahler verwendet werden.

Zur Erhöhung des Umsatzes bzw. um die Spezifität der Reaktion weiter zu erhöhen, kann monochromatische Strahlung verwendet werden und werden hierzu insbesondere Laser eingesetzt.

Wie bereits erwähnt, wird besonders bevorzugt das Vernetzungsverfahren zur Herstellung eines medizinischen Handschuhs oder eines Operationshandschuhes angewandt. Insbesondere vorteilhaft kann hiermit auch ein nitrosaminfreier Handschuh, insbesondere aus Naturkautschuk, hergestellt werden.

Der Fotoinitiator und/oder Spaltprodukte aus der Reaktion können kovalent an die Elastomermoleküle gebunden werden, wodurch der Effekt einer möglichen Migration dieser Moleküle aus dem Handschuh weiter verringert werden kann.

Ebenso ist es möglich, dass an den Elastomermolekülen zumindest ein Vernetzungshilfsmittel, insbesondere ein multifunktionelles Thiol, immobilisiert, insbesondere kovalent gebunden, ist, um die Gefahr einer Hautreizung bei der Verwendung eines Handschuhs weiter zu minimieren.

Der erfindungsgemäße Handschuh kann nitrosaminfrei und/oder beschleunigerfrei und/oder schwefelfrei hergestellt werden, wobei schwefelfrei in dem Sinne zu verstehen ist, dass kein freier Schwefel, wie er für die Schwefelvernetzung verwendet wird, vorhanden ist.

Zum besseren Verständnis der Erfindung wird diese auch anhand der nachfolgenden Figuren näher erläutert.

Es zeigen jeweils in schematisch stark vereinfachter Darstellung:

Fig. 1 einen Ablauf zur Herstellung von gepuderten Operationshandschuhen;

Fig. 2 eine erfindungsgemäße Vorrichtung zur Vorvernetzung mit zweifachem Belichtungszyklus;

Fig. 3 die Anordnung mehrerer Bestrahlungseinheiten für die Nachvernetzung des Latex auf der Form;

Fig. 4 eine Ausführungsvariante der Anordnung der Bestrahlungseinheiten für die Nachvernetzung;

Fig. 5 eine Ausführungsvariante der Anordnung der Bestrahlungseinheiten für die Nachvernetzung;

Fig. 6 eine Ausführungsvariante der Anordnung der Bestrahlungseinheiten für die Nachvernetzung;

Fig. 7 ein Diagramm der Reißfestigkeit des Elastomers in Abhängigkeit von der Anzahl der Belichtungszyklen vor einer Heißluftalterung;

Fig. 8 ein Diagramm der Reißfestigkeit des Elastomers in Abhängigkeit von der Anzahl der Belichtungszyklen nach einer Heißluftalterung.

Es sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1 oder 5,5 bis 10.

Fig. 1 zeigt eine Ausführungsvariante eines schematischen Ablaufs zur Herstellung von Operationshandschuhen aus einem Latex durch Tauchen in einer Anlage 1.

Es sei daraufhingewiesen, dass die Erfindung nicht auf die Herstellung von Operationshandschuhen beschränkt ist, sondern vielmehr generell auf vernetzte Elastomere gerichtet ist, insbesondere die voranstehend erwähnten Produkte. Neben Handschuhen können auch andere Latexprodukte erfindungsgemäß hergestellt werden, wie beispielsweise Kondome, diverse medizinische Latexprodukte, wie beispielsweise Katheter, Diaphragmen, Infusionsbeutel, medizinische Schläuche, Gewebekulturgefäße, etc. oder aber auch Latexprodukte des Konsumgüterbereichs, wie z.B. Schwimmflossen, Schnuller, etc.

In einem Mischschritt 2 werden die für einen Vorvulkanisationsschritt 3 erforderlichen Chemikalien dem Latex beigemischt und erfolgt gegebenenfalls eine Homogenisierung des Latex. Der compoundierte Latex wird in der Folge im Vorvulkanisationsschritt 3 vorvulkanisiert. In der Folge wird der vorvulkanisierte Latex in eine Kettentauchanlage 4 überführt, wo er bzw. das Halbfertigfabrikat die Schritte Tauchen 5, Rändern 6, Nass-Leaching 7, Trocknen 8, gegebenenfalls Trocken-Leaching 9, gegebenenfalls Pudern 10, Abziehen 11, Verpackung 12, Qualitätskontrolle 13 und gegebenenfalls Sterilisation 14 durchläuft. Die Tauchformen werden vor dem erneuten Tauchen in Latex einer Reinigung 15, einer Koagulanttauchung 16 und einer Trocknung 17 unterzogen.

Die Tauchformen bestehen üblicherweise aus Porzellan, können aber auch aus Glas, Edelstahl oder Kunststoff angefertigt sein. Eine saubere Oberfläche dieser Tauchform ist ein Kriterium für eine homogene Abscheidung des Latexfilms im anschließenden Tauchprozess. Sowohl basische als auch saure Lösungen, oxidierende Verbindungen, Tenside oder auch häufig eine Kombination dieser Reinigungschemikalien wird für die Entfettung und Säuberung der Tauchformen eingesetzt.

Die Zusammensetzung des Koagulationsbades ist unter anderem ein Parameter für die Schichtdicke des abgeschiedenen Latexfilms. Das Koagulationsbad setzt sich aus dem Koagulanten (üblicherweise $CaNO_3$, wahlweise auch $CaCl_2$), dem Trennmittel ($CaCO_3$) und dem Benetzungsmittel (kationische Tenside) zusammen. Das Trennmittel

erleichtert das Abziehen des Handschuhs von der Tauchform, wobei in einigen puderfreien Prozessen andere anorganische Salze und zum Teil auch Polymere verwendet werden können, wie dies aus dem Stand der Technik bekannt ist.

Die abgeschiedenen positiven Metallionen auf der Oberfläche der Tauchform bewirken eine Entladung und in weiterer Folge die Koagulierung des negativ stabilisierten NR-Latex, sobald die Form in den vorvernetzten Latex taucht. Abhängig von der Eintauchzeit und der Konzentration der Metallionen werden unterschiedliche Filmdicken erhalten.

Handschuhe werden mit einem gerollten Rand am unteren Schaftende hergestellt. Zu diesem Zweck wird ein Teil des abgeschiedenen Films beim Rändern 6 durch rotierende Bürsten mechanisch zusammen gerollt. Durch die Klebrigkeit des Films bleibt der gerollte Wulstrand über den gesamten Herstellungsprozess bestehen.

Dem nassen Latexfilm wird durch eine kurze Antrocknung mechanische Festigkeit verliehen bevor das Nass-Leaching 7 erfolgt. Durch das Tauchen der Latexfilme in ein warmes Wasserbad werden neben dem Koagulanten ($CaNO_3$ / $CaCl_2$) auch Proteine zumindest teilweise ausgewaschen.

Zur Herstellung puderfreier Handschuhe kann anstelle des Puderns 10 eine Oberflächenbehandlung, z.B. durch Chlorierung, erfolgen, um die An- und Ausziehbarkeit der Handschuhe zu verbessern. Es sind aber auch Gleitbeschichtungen möglich.

Da Tauchverfahren für Handschuhe an sich aus dem Stand der Technik bekannt sind, sei der Fachmann beispielsweise auf die EP 0 856 294 A, insbesondere die Fig. 4 und 5 dieser EP-A sowie die zugehörigen Ausführungen in Spalte 14, Zeile 38 bis Spalte 18, Zeile 51 verwiesen, insbesondere in Hinblick auf die Ausführungen bezüglich der Koagulation, des Tauchens in Latex, diverser Waschvorgänge, diverser Nachbehandlungen, wie z.B. Chlorieren bzw. Halogenieren der Oberfläche der Handschuhe bzw. des Latex, die Herstellung von Oberflächenrauhigkeiten bzw. die Bereitstellung von puderfreien Handschuhen, etc.. Es soll damit auf unnötige Wiederholungen des Standes der Technik im Zusammenhang mit vorliegender Erfindung verzichtet werden und bildet daher die EP 0 856 294 A1 zumindest im besagten Umfang einen Teil der Offenbarung gegenständlicher Anmeldung.

Fig. 2 zeigt eine bevorzugte Ausführungsvariante einer Vorvernetzungseinrichtung 18. Diese ist in der Anlage nach Fig. 1 zwischen dem Mischschritt 2 und dem Tauchen 5 angeordnet, wobei die Vorvernetzungseinrichtung 18 den Mischschritt 2 auch umfassen kann.

Bei dieser Variante umfasst die Vorvernetzungseinrichtung 18 zwei Reaktoren 19, 20, insbesondere Fallfilmreaktoren, die in Flussrichtung des Latex -Pfeil 21 - hintereinander geschaltet sind, sodass der Latex zwei Belichtungszyklen in der Vorvernetzung durchläuft, also zweimal der Bestrahlung mit UV- und/oder VIS-Licht ausgesetzt ist. In den Reaktoren 19, 20 sind dazu Strahlungsquellen 22, 23 angeordnet, insbesondere Hg-Hochdruckdampflampen. Vor und/oder zwischen und/oder nach den Reaktoren 19, 20 können weitere Behälter angeordnet sein, beispielsweise drei Behälter 24, 25 und 26 wie im Fall der Ausführungsvariante nach Fig. 2. Diese Behälter können beispielsweise der Zudosierung von (weiteren) Prozesschemikalien, beispielsweise dem Fotoinitiator und/oder Covernetzer, dienen oder als Zwischenlagerbehälter für den gemischten bzw. vorvernetzten Latex. Des Weiteren besteht die Möglichkeit, dass in diesen Behälter(n) 24, 25 der Fotoinitiator in einer Voremulsion bzw. Vordispersion, gegebenenfalls mit einem entsprechenden Emulsionsmittel, vorgelegt wird. Als Emulsionsmittel können dazu beispielsweise Tenside, wie bereits erwähnt, verwendet werden.

Weiters kann zumindest eine Fördereinrichtung 27 vorgesehen sein, mit der der Latex durch die Vorvernetzungseinrichtung 18 gefördert wird. In der Ausführung nach Fig. 2 ist hierzu eine zweite Fördereinrichtung 28 vorgesehen, die in Flussrichtung - Pfeil 21 - zwischen den beiden Reaktoren 19, 20 angeordnet ist. Beispielsweise können die Fördereinrichtungen 27, 28 durch Exzenterschneckenpumpen gebildet werden.

Zur Steuerung und/oder Regelung können weiters entsprechende Regler, Ventile, etc. an den entsprechenden Stellen angeordnet werden.

Diese Reaktorkaskade nach Fig. 2 hat den Vorteil, dass die zweistufige Vorvernetzung des Latex kontinuierlich erfolgen kann. Es ist aber auch möglich, diese Vorvernetzung mit nur einem Reaktor 19 durchzuführen, wozu der Latex dann im Kreislauf geführt werden muss, um eine mehrmalige Belichtung zu ermöglichen.

Anstelle von Fallfilmreaktoren können beispielsweise auch Tauchreaktoren verwendet werden, in die die Strahlungsquelle 23 eintaucht.

Fig. 3 zeigt schematisch eine Ausführungsvariante einer Nachvernetzungseinrichtung 29. Dargestellt sind zwei Tauchformen 30, 31 sowie sechs Bestrahlungseinheiten 32 bis 37. Bevorzugt ist diese Nachvernetzungseinheit 29 im Bereich desRänderns 6 der Anlage 1 (Fig. 1) angeordnet, da im Falle der Herstellung von Handschuhen der translatorischen Vorwärtsbewegung - Pfeil 38 - bei dieser Anordnung eine Rotationsbewegung - Pfeil 39 - überlagert ist, wodurch die Bestrahlung einheitlicher erfolgen kann, insbesondere "abgeschattete" Bereiche bei den Fingern besser ausgeleuchtet werden können.

Generell ist anzumerken, dass die Nachvernetzungseinrichtung 29 nicht zwingend an dieser Stelle in der Anlage 1 (Fig. 1) angeordnet werden muss, sondern auch an einer anderen Stelle nach dem Tauchen 5 bzw. der Formgebung des Latex platziert werden kann. Es müssen auch nicht sechs Bestrahlungseinheiten 32 bis 37 verwendet werden, sondern kann auch nur eine Bestrahlungseinheit 32 Anwendung finden, insbesondere bei Produkten mit einfacher Geometrie, beispielsweise bei ebenen Filmen, oder eine von sechs verschieden Anzahl, beispielsweise zwei, drei, vier,

fünf, sieben, etc.

Bevorzugt werden als Bestrahlungseinheiten 32 bis 37 aus voranstehend genanntem Grund wieder Ga-dotierte Hg-Hochdruckdampflampen verwendet. Es können aber auch andere UV- bzw. UV/VIS-Strahler, beispielsweise die voranstehend genannten, eingesetzt werden.

Für den Fall, dass die Nachvernetzungseinrichtung 29 an einer anderen Stelle in einer Produktionsanlage von vernetzten Elastomer(produkten) situiert wird, besteht bei Bedarf auch die Möglichkeit der Anordnung einer zusätzlichen Dreheinrichtung, um die Bestrahlung zu homogenisieren.

Bevorzugt ist vor dem Tauchen 5 und nach der Vorvernetzungseinrichtung 18 in der Anlage 1 (Fig.) eine Trockeneinheit, beispielsweise eine Trockenkammer oder ein Heißluftofen, angeordnet (nicht dargestellt), da sich im Zuge der Entwicklung der Erfindung herausgestellt hat, dass höhere Reißfestigkeiten erzielt werden, wenn der Gehalt an Restfeuchte maximal die voranstehend angeführten Werte annimmt.

Die Fig. 4 bis 6 zeigen verschiedene Möglichkeiten der Bestrahlung von mit einem Latexfilm versehenen Tauchformen 30. So ist in Fig. 4 eine Bestrahlung im rechten Winkel gezeigt, wohingegen die Fig. 5 und 6 eine Bestrahlung in einem Winkel 40 von 30 ° zeigt. Zur Ausbildung des Winkels 40 wird die Tauchform 30 entsprechend gegen die Bezugsebene (die Horizontale im Beispiel nach den Fig. 5 und 6) geneigt.

Anhand von Dosismessstreifen, die an unterschiedlichen Stellen der Handform angeordnet wurden (sowohl an den Fingerzwischenräumen als auch an der Handfläche), konnte gefunden werden, dass bereits bei einer simplen Belichtung senkrecht auf die Handform (Fig. 4) auch kritische Bereiche ausgeleuchtet werden können, wobei die Dosisverteilung in diesem Fall im Bereich von 1:6 liegt (siehe Tabelle 1). Durch eine 30° Neigung der Handform zum UV-Strahler kann die Dosisverteilung deutlich verringert werden (liegt im Bereich 1:3 bis 1:4).

Tabelle 1

| Position 1 | | | | | |
|---|---|---|---|---|---|
| Messpunkte | 1 | 2 | 3 | 4 | 5 |
| Dosis [$mJ/cm^2$] | 30 | 30 | 30 | 20 | 120 |
| plus Position 2 | | | | | |
| Messpunkte | 1 | 2 | 3 | 4 | 5 |
| Dosis [$mJ/cm^2$] | 100 | 100 | 30 | 30 | 200 |
| plus Position 3 | | | | | |
| Messpunkte | 1 | 2 | 3 | 4 | 5 |
| Dosis [$mJ/cm^2$] | 100 | 100 | 70 | 70 | >200 |

Analoges wurde für den Handrücken gefunden.

Es ist also bei geometrisch komplexeren Formen von Vorteil, wenn mehr als eine Strahlungsquelle verwendet wird, wobei die Strahlungsquellen in unterschiedlichen Winkeln - abhängig von der Geometrie - zur Form angeordnet werden und/oder wenn die Form eine Rotationsbewegung durch die Strahlung durchführt, wobei auch in diesem Fall der Neigungswinkel der Form zur Strahlungsquelle bedarfsweise während der Bestrahlung verändert werden kann.

Mit Hilfe der Strahlungsquellen 22, 23 bzw. der Bestrahlungseinheiten 32 bis 37 wird Energie in Form von Photonen auf den Fotoinitiator, d.h. dessen Moleküle, übertragen und kann dadurch eine radikalische Spaltung der Moleküle des Fotoinitiators ausgelöst werden. Mit Hilfe der dabei entstehenden Radikale können in der Folge Doppelbindungen, welche in den Latexmolekülen vorhanden sind, beispielsweise in der Hauptkette und/oder in einer Seitenkette, entsprechend dem radikalischen Reaktionsmechanismus aufgebrochen werden und somit die Vernetzung über Startermoleküle oder direkt zwischen den Elastomermolekülen erfolgen.

Daneben besteht nach einer weiteren Ausführungsvariante die Möglichkeit, der Polymerdispersion und/oder Voremulsion zumindest einen Hilfsstoff zuzusetzen. Dieser Hilfsstoff kann beispielsweise ein Vernetzungshilfsmittel (Covernetzer) sein. Beispielsweise besteht die Möglichkeit als Vernetzungshilfsmittel ein Thiol und/oder ein Selenol vorzulegen. Bevorzugt weist das Thiol zwei oder mehrere SH-Gruppen auf, wodurch die Möglichkeit der so genannten Thiol-en Additionsreaktion, um damit Vernetzungsstellen im Polymer zu schaffen, zur Verfügung steht. Es kann damit eine Beschleunigung der Vernetzungsreaktion erreicht werden. Neben diesem Vernetzungshilfsmittel können auch weitere Vernetzungshilfsmittel bzw. weitere Hilfsstoffe, wie z.B. Sensibilisatoren, Wasserstoffdonoren, diverse Prozessadditiva, wie z.B. Stabilisatoren, Antischaummittel, Dispergiermittel, Emulgatoren, Koaguliermittel, Vernetzungschemikalien, Farbstoffe und auch Füllstoffe in der Polymerdispersion bzw. zumindest teilweise in der Voremulsion vorhanden sein, wobei diese Reagenzien zumindest großteils aus dem Stand der Technik bekannt sind und sei der Fachmann an

dieser Stelle an die einschlägige Literatur diesbezüglich verwiesen, beispielsweise die EP 0 856 294 A1 der Anmelderin bzw. auf die Veröffentlichung "Kautschuktechnologie" (Röthemeyer/Sommer, Carl Hanser Verlag 2001).

Die Voremulsion bzw. Vordispersion kann zumindest teilweise der Polymerdispersion vor dem Start der Vorvernetzungsreaktion und/oder der Nachvernetzung zugesetzt werden. Ebenso ist es möglich diese zumindest teilweise während der Reaktion dieser Polymerdispersion beizufügen, beispielsweise in kleinen Mengen zuzugeben, beispielsweise zuzutropfen.

Die Strahlungsquellen 22, 23 und die Bestrahlungseinheiten 32 bis 37 können Licht im bereits beschriebenen Spektralbereich, also insbesondere zwischen 200 nm und 550 nm bzw. 250 nm und 475 nm bzw. 275 nm und 400 nm, aussenden.

Hinsichtlich des Fotoinitiators bzw. möglicher Mischungen verschiedener Fotoinitiatoren sei an dieser Stelle auf voranstehende Ausführungen verwiesen.

Sowohl die Strahlungsquellen 22, 23 und die Bestrahlungseinheiten 32 bis 37 als auch diverse Rührwerke bzw. gegebenenfalls weitere Komponenten der Anlage 1 können mit einer Steuer- und/oder Regeleinrichtung (nicht dargestellt), wie z.B. einem PC oder generell einer Datenverarbeitungsanlage, wirkungsverbunden sein, sodass gegebenenfalls eine Automatisierung bzw. ein Prozessablauf mit veränderlichen Reaktionsparametern vollautomatisch durchgeführt werden kann. Um dazu beispielsweise die Temperatur zu verändern, kann an oder in dem oder den Reaktor(en) 19, 20 eine entsprechende Heiz- und/oder Kühleinrichtung aus dem Stand der Technik angeordnet werden.

Es ist des Weiteren möglich, die Vorvernetzung und/oder Nachvernetzung bei einem Druck, der unterschiedlich zum Atmosphärendruck ist, durchzuführen, beispielsweise bei Unterdruck, ebenso ist es möglich, die Vernetzungsreaktion bei Überdruck auszuführen und kann dazu zumindest einer der Reaktoren 19, 20 oder die Nachvernetzungseinrichtung 29 diesbezüglich entsprechend ausgestaltet werden, also beispielsweise vakuumdicht oder aber auch geeignet um Überdruckreaktionen durchzuführen, also beispielsweise mit verstärkten Wandungen.

Der Fallfilmreaktor weist an zumindest einer Oberfläche entsprechende Vorkehrungen, wie z.B. Sichtfenster, auf, um das Eindringen der elektromagnetischen Strahlung in das Innere des Reaktors bzw. der Reaktoren 19, 20 zu ermöglichen. Des Weiteren ist es möglich diesen aus einem UV-durchsichtigen Werkstoff, wie z.B. (Quarz)Glas oder Kunststoff, zumindest teilweise zu bilden.

Die Anordnung der Strahlungsquellen 22, 23 kann aber auch im Inneren des Fallfilmreaktors erfolgen, sodass also die Polymerdispersion von innen heraus belichtet wird

Selbstverständlich ist es möglich, dass die Vorvernetzung und/oder Nachvernetzung des Latex bzw. der Latices gesondert, d.h. unabhängig von der weiteren Produktionsanlage für Latexprodukte, durchgeführt wird.

Hinsichtlich der verwendeten Fotoinitiatoren, der diversen Hilfsstoffe sowie deren Konzentrationen bzw. Anteile soll ebenso wie für die einsetzbaren Latices und deren Anteil an der Polymerdispersion generell zur Vermeidung unnötiger Wiederholungen auf obige Ausführungen an dieser Stelle verwiesen sein.

Eingesetzte Feststoffe können im Rahmen der Erfindung in einem Solvens gelöst werden und/oder in Wasser emulgiert bzw. dispergiert werden.

Es ist im Rahmen der Erfindung weiters möglich, nach der Formgebung eine Nachbehandlung durchzuführen, z.B. durch Erwärmen, Belichten oder Extrahieren.

Die Reißfestigkeiten der im Rahmen der Erfindung hergestellten Latexfilme können im Bereich von 25 $N/mm^2$ bis 30 $N/mm^2$, insbesondere für Naturkautschuk, liegen bei einer Reißdehnung von 800 % - 900 %.

Es ist weiters möglich die strahlenchemische Vernetzung sowohl kontinuierlich als auch diskontinuierlich durchzuführen.

Im Folgenden sind einige Beispiele angegeben, die im Rahmen der Erfindung durchgeführt worden sind. Die Anführung aller durchgeführten Versuche würde allerdings den Rahmen vorliegender Beschreibung sprengen. Die Beschränkung auf die Angabe der folgenden Beispiele bedeutet daher nicht, dass die Erfindung darauf beschränkt ist. Vielmehr bewegt sich die Erfindung bezüglich der verwendeten Latices bzw. Chemikalien und deren Anteile im voranstehend abgesteckten Rahmen.

Es wurden folgende Materialien bzw. Chemikalien verwendet:

| Materialien | Nähere Bezeichnung |
|---|---|
| NR-Latex | high ammonia NR-Latex / 60 % drc. |
| IR-Latex | Kraton® IR-401 Latex / 60 % drc. |
| Koagulationsbad | $CaCl_2$-Lösung (10 Gew.-%) in Wasser / Zusätze: Kreide, Benetzungsmittel |
| Gleitbad | Mischung aus Silikon-, Acrylat- und Polyurethanbestandteilen |

(fortgesetzt)

| Chemikalie | Hersteller | Strukturformel |
|---|---|---|
| Fotoinitiatoren | | |
| Genocure DMHA | Rahn AG | 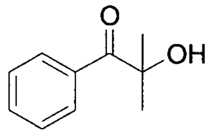 |
| Irgacure 2959 | Rahn AG | 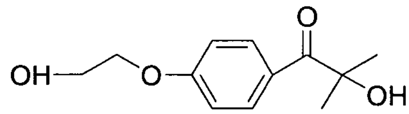 |
| Genocure MBF | Rahn AG | 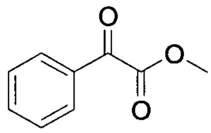 |
| Lucirin TPO L | BASF | 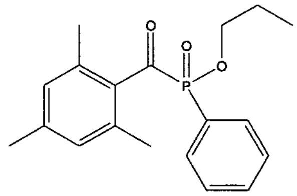 |
| | | |
| Covernetzer | | |
| Trimethylolpropantris-3-mercaptopropionat | Bruno Bock Thiochemicals | |
| | | 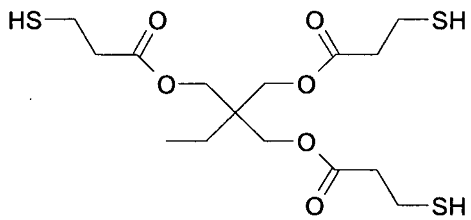 |
| Pentaerythritol tetrakis-3-mercaptopropionat | Bruno Bock Thiochemicals | 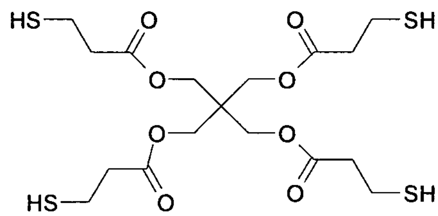 |
| Sonstige Chemikalien | | |
| Tween 20 | Sigma-Aldrich | 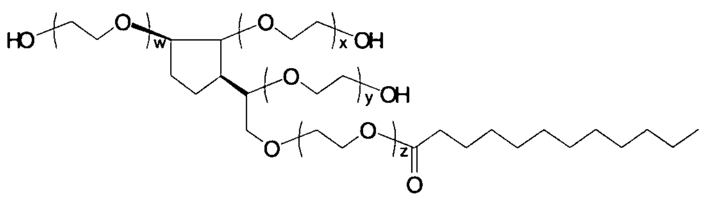 |
| Ralox LC® | Solvadis | 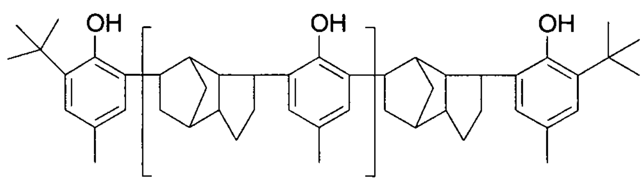 |

(fortgesetzt)

| | | |
|---|---|---|
| Sonstige Chemikalien | | |
| Nekal BX | BASF | 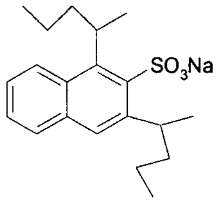 |
| Ascorbylpalmitat | Sigma-Aldrich | 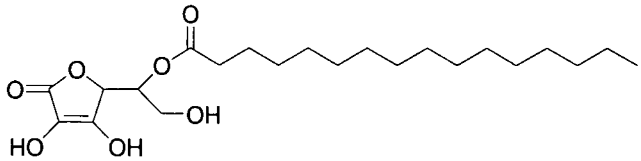 |
| Linolsäure | Sigma-Aldrich | 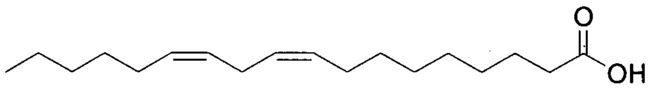 |
| α-Tocopherol | Sigma-Aldrich | 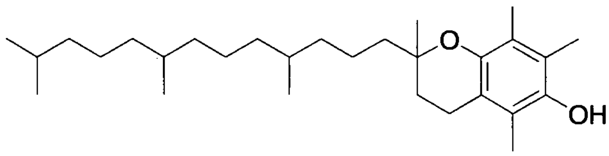 |

Beispiel 1: UV-Vernetzung von Naturkautschuk (NR-Latex)

UV-Vorvernetzung im Fallfilmreaktor:

(Die Angabe phr steht für parts per hundred parts rubber)

| Prozessparameter | | Prozessparameter | |
|---|---|---|---|
| Strahlerleistung (Hg-Hochdrucklampe) [W] | 3000 | Fördergeschwindigkeit [l/min] | 1,28 |
| Anzahl der Belichtungszyklen | 2 | Schichtdicke (Fallfilm) [mm] | 0,45 - 0,6 |
| Fotoinitiator | Genocure DMHA (0,5 - 1,0 phr) | Feststoffgehalt (Latex) [drc.] | 40 |
| Thiol-Vernetzer | Tris-Thiol (1,0 phr) | Kühlwasserdruck [bar] | 0,6 |

| Tauchung: | | | |
|---|---|---|---|
| Prozessparameter | | Prozessparameter | |
| Lagerzeit flüssiger Latex (vorvernetzt) [Tage] | 0-1 | Trocknungstemperatur [°C] | 120 |
| Stabilisator | Ralox LC (0,5 phr) | Trocknungsdauer [min] | 20 |

| Nachdosierung vor der Tauchung Prozessparameter | | Prozessparameter | |
|---|---|---|---|
| Fotoinitiator | Genocure DMHA (1,0 - 1,4 phr) | Thiol-Vernetzer | Tetra-Thiol (1,0 phr) |
| Nachvernetzung Prozessparameter | | | |

(fortgesetzt)

| Nachdosierung vor der Tauchung Prozessparameter | | Prozessparameter | |
|---|---|---|---|
| Bestrahlungsdosis [J/cm²] | ~5 | | |

Im Zuge der Vernetzungsexperimente wurde NR-Latex bei einer Strahlerleistung von 3000 W einfach, zweifach und dreifach vorvernetzt, wobei nach jedem Belichtungszyklus Latexproben direkt am Ausflussventil entnommen worden sind. Die Ergebnisse sind in den Fig. 7 und Fig. 8 dargestellt. Es wurden jeweils sterile Proben (Quadrate) und nicht sterile Proben (Dreiecke) vermessen. Die Reißfestigkeiten in $N/mm^2$ (Ordinate) sind in den Fig. gegen die Anzahl der Belichtungen (Abszisse) aufgetragen.

Aus den Ergebnissen kann geschlossen werden, dass nach einer zweimaligen Vorvernetzung ein Maximum in den mechanischen Festigkeiten (Vernetzungsdichte liegt bei 0,28 $mmol/cm^3$) erzielt wird. In diesem Fall weisen sterile, nicht gealterte NR-Latexfilme (Lagerung 7 Tage bei 20 °C) eine Reißfestigkeit von 26,5 $N/mm^2$ auf, während nach der Heißluftalterung die mechanischen Eigenschaften auf 22,0 $N/mm^2$ sinken.

Bei einem einmaligen Belichtungszyklus ist die Vernetzungsdichte (0,19 $mmol/cm^3$) anscheinend zu gering, sodass die anschließend getauchten Latexfilme weitaus geringere Reißfestigkeiten (21 $N/mm^2$) verbunden mit einer schlechteren Alterungsbeständigkeit (13 - 15 $N/mm^2$) aufwiesen.

Nach einem dritten Belichtungsdurchgang wird ebenfalls eine Verringerung der Reißfestigkeiten beobachtet, da es in Folge einer Übervernetzung (Vernetzungsdichte liegt bei 0,49 $mmol/cm^3$) ebenfalls zu einer Abnahme der mechanischen Festigkeiten kommt, wobei die Alterungsbeständigkeit eines übervernetzten NR-Latexfilms weitaus höher ist im Vergleich zu einem untervernetzten (ein Belichtungszyklus).

Es wurde weiters gefunden, dass eine Vernetzung (Vorvernetzung und/oder Nachvernetzung) unter Inertgasatmosphäre (Argon oder Stickstoff) nach dem zweiten oder dritten Belichtungszyklus zu einer Erhöhung der mechanischen Festigkeiten von bis zu 5 $N/mm^2$ führt.

Auch eine Trocknung der Latexfilme bei Raumtemperatur anstelle bei erhöhter Temperatur kann zu Reißfestigkeiten führen, die um bis zu 5 $N/mm^2$ höher sind.

Durch die Nachvernetzung konnten schließlich Reißfestigkeiten bis zu 33 $N/mm^2$ erreicht werden, wobei bei einer Strahlungsdosis über 5 $J/cm^2$ bereits erste Abbaureaktionen in Verbindung mit Luftsauerstoff beobachtet werden konnten, die zu einer Verringerung der Reißfestigkeit führten.

Beispiel 2: UV-Vernetzung von IR-Latex

| UV-Vorvernetzung im Fallfilmreaktor Prozessparameter | | Prozessparameter | |
|---|---|---|---|
| Strahlerleistung (Hg-Hochdrucklampe) [W] | 800 | Fördergeschwindigkeit [l/min] | 1,28 |
| Anzahl der Belichtungszyklen | 2 | Schichtdicke (Fallfilm) [mm] | 0,45 - 0,6 |
| Fotoinitiator | Lucirin TPO L (0,5 - 1,0 phr) | Feststoffgehalt (Latex) [drc.] | 40 |
| Thiol-Vernetzer | Tetra-Thiol (0,2 - 1,0 phr) | Kühlwasserdruck [bar] | 0,6 |

| Tauchung Prozessparameter | | Prozessparameter | |
|---|---|---|---|
| Lagerzeit flüssiger Latex (vorvernetzt) [Tage] | < 1 | Restfeuchtegehalt [%] | <4 |
| Alterungsschutz[1] | Ralox LC (0,5 phr) | Stabilisator | Nekal BX (1,25 phr) |

(fortgesetzt)

| Nachdosierung vor der Tauchung Prozessparameter | | Prozessparameter | |
|---|---|---|---|
| Fotoinitiator | Lucirin TPO L (1,0 - 1,5 phr) | Thiol-Vernetzer | Tetra-Thiol (0,9- 1,4 phr) |

| Nachvernetzung Prozessparameter | |
|---|---|
| Bestrahlungsdosis [J/cm$^2$] | ~ 4 - 13 |
| UV-Lichtquelle | Ga-dotierter Hg-Strahler |
| 1...wahlweise auch 0,5 phr α-Tocopherol drc.... Feststoffgehalt | |

Generell sei angemerkt, dass die Strahlerleistung im Zuge der Erfindung in der Vorvernetzungstufe zwischen 300 W und 3000 W, insbesondere zwischen 400 W und 1000 W, jene in der Nachvernetzungsstufe zwischen 2500 W und 4500 W, insbesondere zwischen 3000 W und 3500 W betragen hat, wobei es von Vorteil ist, wenn die Vorvernetzung mit einer geringeren Leistung durchgerührt wird. Insbesondere bei IR-Latices ist es von Vorteil, wenn die Strahlerleistung der Vorvernetzung annähernd 800 W nicht übersteigt, um eine bessere Filmbildung zu erhalten. Es sei jedoch angemerkt, dass die verwendete Strahlerleistung in Abhängigkeit der Geometrie der Bestrahlungsanlage von den beispielhaft angegebenen Werten abweichen kann.

Auch der Zusatz von Tensiden in der Vorvernetzungsstufe, insbesondere Tenside mit ungesättigten C-C Bindungen, wie z.B. Linolsäure, kann die Filmbildung verbessern.

Weiters konnte insbesondere bei IR-Latices die Filmbildung durch die Zugabe von Radikalfängern, wie z.B. Ascorbylpalmitat (Anteil 0,25 phr bis 2 phr, insbesondere 0,5 phr bis 1 phr), verbessert werden, sodass auch nach der Trocknung rissfreie Filme erhalten wurden. Ascorbylpalmitat hat dabei die Eigenschaft sich an die Oberfläche der Latexpartikel anzulagern.

Es ist auf Grund des Allergiepotentials der Produkte und der Wirtschaftlichkeit des Verfahrens ein möglichst geringer Chemikalieneinsatz erstrebenswert, aber für hohe mechanische Festigkeiten (> 20 N/mm$^2$) werden zumindest 1,0 phr Genocure DMHA und 1,0 phr Tetra-Thiol bevorzugt. Diese Reißfestigkeiten konnten in Abwesenheit eines Stabilisators verbessert werden.

Ralox LC z.B. als substituiertes BHT-Derivat fängt hierbei sehr wirkungsvoll die Startradikale in der Postvulkanisation, wodurch die Thiol-en Vernetzung vor allem bei einer niedrigen Konzentration (Nachdosierung von je 1,0 phr Genocure DMHA und 1,0 phr Tetra-Thiol) der Prozesschemikalien inhibiert wird.

Die Verwendung von Lucirin TPO L als Fotoinitiator bringt den Vorteil mit sich, dass durch die Absorption bis in den sichtbaren Wellenlängenbereich einerseits die Strahleremission effizienter für die Nachvernetzung eingesetzt werden kann und andererseits das langwellige Licht in tiefere Schichten vordringen kann, wodurch eine homogenere Vernetzung über den Latexfilm erreicht werden kann.

Eine Verbesserung der Alterungsbeständigkeit von UV vernetzten (Vor- plus Nachvernetzung) IR-Latexfilmen konnte mit α-Tocopherol (Vitamin E), das als essentielle Verbindung im Stoffwechsel des menschlichen Körpers vorkommt, und daher hinsichtlich der allergologischen Verträglichkeit vorteilhaft ist, erreicht werden. Der Anteil an Vitamin E kann zwischen 0,2 phr und 1 phr betragen.

Die Zugabe von Vitamin E als Stabilisator ist im Rahmen der Erfindung jedoch nicht auf IR-Latices beschränkt. Vitamin E kann generell für diesen Zweck auch in anderen Latices verwendet werden.

Vitamin E hat zudem den Vorteil, dass es - überraschenderweise - die Thiol-en Reaktion nicht oder nur geringfügig inhibiert.

Zur Verbesserung der Stabilität der Latexmischung kann beispielsweise ein Naphthylsulfonat (Nekal BX; 0,2 phr - 2,5 phr) zugesetzt werden, wodurch die Förderbarkeit des Latex durch geringere Ablagerungen in der Anlage 1 deutlich verbessert werden kann.

Weiters ist es von Vorteil, wenn der Vorvernetzungsgrad zwischen 80 % und 120 %, insbesondere zwischen 80 % und 90 % beträgt. Dazu wird die Netzstellendichte zur Abschätzung des Vernetzungsgrades bestimmt, da diese ein schnell zu bestimmender Parameter ist, um den Reaktionsfortschritt der fotochemischen Vernetzung zu charakterisieren.

Die Netzstellendichte liefert exaktere Aussagen über die Vernetzungsdichte bzw. das mittlere Molekulargewicht der Polymerketten zwischen den einzelnen Vernetzungsstellen. Dazu wird der Quellungsgrad der UV vernetzten Latexfilme mit Hilfe der Flory-Rehner Methode bestimmt.

Ca. 60 mg des vernetzten Latexfilmes, werden in 3 ml Toluol für 48 h bei 30°C im Trockenschrank gequollen. Anschließend werden die Filme über ein Filterpapier filtriert und mehrmals in Diethylether getaucht. 30 s nach dem Entfernen der Probe aus dem Diethylether wird der Latexfilm gewogen (+/- 0,5 mg) und die Probe bei 70°C im Trockenschrank bis zur Gewichtskonstanz getrocknet und erneut gewogen. Die Vernetzungsdichte wird anschließend bei Kenntnis des Flory-Huggins Wechselwirkungsparameter zwischen Lösungsmittel und Polymer mit nachfolgendem Gleichungssystem bestimmt.

$$\overline{M_C} = -V_l \rho_p \frac{\phi_p^{1/3} - \frac{\phi_p}{2}}{\ln(1-\phi_p) + \phi_p + \chi_1 \phi_p^2}$$

$$\frac{1}{\phi_p} = 1 + \frac{W_s}{W_p} \frac{\rho_P}{\rho_s}$$

| | |
|---|---|
| $\overline{M_C}$ | mittlere molare Masse der Polymerketten zwischen den Vernetzungsstellen |
| $V_l$ | molares Volumen des Lösungsmittels |
| $\phi_p$ | Volumenanteil des Polymers |
| $\chi_1$ | Flory-Huggins Wechselwirkungsparameter zwischen Lösungsmittel und Polymer |
| $W_s$ | Masse des absorbierten Lösungsmittels |
| $W_p$ | Masse des trockenen Polymers |
| $\rho_s$ | Dichte des Lösungsmittels |
| $\rho_p$ | Dichte des Polymers |

Die Bestimmung der mechanischen Festigkeiten der Latexfilme wird in Anlehnung an den ASTM-Standard D412-98a (Annu. Book ASTM Stand. 09.01 (2002)) durchgeführt. Als charakteristischer Parameter wird die Reißfestigkeit herangezogen. Für die mechanische Zugprüfung werden aus einem Latexfilm 3 - 4 Schulterstäbe (Stegbreite: 3 mm) ausgestanzt. Die Dicke des Steges wird mit einer Mikrometerschraube (arithmetischer Mittelwert aus 10 Messwerten) bestimmt und manuell in die Messsoftware eingetragen. Zur Auswertung der Reißfestigkeit wird der arithmetische Mittelwert der Schulterstäbe einer Probe herangezogen.

Zur Bestimmung der Beständigkeit der Elastomeren während der Sterilisation wurden diese mit einer Co60-Quelle bei einer Dosis von 25 kGy bestrahlt. Nach Erhalt der gammasterilisierten Latexfilme werden die Reißfestigkeiten sowohl vor als auch nach der Heißluftalterung (7 Tage bei 70 °C, gemäß EN 455/2) mit der Zugprüfmaschine bestimmt.

Die Ausführungsbeispiele zeigen mögliche Ausführungsvarianten der Erfindung wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Ordnung halber sei abschließend daraufhingewiesen, dass zum besseren Verständnis des Aufbaus der Vorvernetzungseinrichtung 18 und der Nachvernetzungseinrichtung 29 diese bzw. deren Bestandteile teilweise unmaßstäblich und/oder vergrößert und/oder verkleinert dargestellt wurden.

**Bezugszeichenaufstellung**

| | |
|---|---|
| 1 | Anlage |
| 2 | Mischschritt |
| 3 | Vorvulkanisationsschritt |
| 4 | Kettentauchanlage |
| 5 | Tauchen |
| 6 | Rändern |

7 Nass-Leaching
8 Trocknen
9 Trocken-Leaching
10 Pudern

11 Abziehen
12 Verpackung
13 Qualitätskontrolle
14 Sterilisation
15 Reinigung

16 Koagulanttauchung
17 Trocknung
18 Vorvernetzungseinrichtung
19 Reaktor
20 Reaktor

21 Pfeil
22 Strahlungsquelle
23 Strahlungsquelle
24 Behälter
25 Behälter

26 Behälter
27 Fördereinrichtung
28 Fördereinrichtung
29 Nachvernetzungseinrichtung
30 Tauchform

31 Tauchform
32 Bestrahlungseinheit
33 Bestrahlungseinheit
34 Bestrahlungseinheit
35 Bestrahlungseinheit

36 Bestrahlungseinheit
37 Bestrahlungseinheit
38 Pfeil
39 Pfeil
40 Winkel

**Patentansprüche**

1. Verfahren zur Herstellung eines vernetzten Elastomers durch Bestrahlung einer Polymerdispersion aus zumindest einem vernetzbaren Polymer mit elektromagnetischer Strahlung im ultravioletten (UV-Licht) und/oder sichtbaren Spektralbereich, wobei die Vernetzung in zumindest zwei Schritten als Vorvernetzung und Nachvernetzung durchgeführt wird und der Polymerdispersion zumindest ein Fotoinitiator zur Auslösung der Vernetzungsreaktion vor der Vorvernetzung zugesetzt wird, **dadurch gekennzeichnet, dass** der vorvernetzten Polymerdispersion vor und/oder während der Nachvernetzung nochmals zumindest ein Fotoinitiator zugesetzt und die Nachvernetzung ebenfalls mit elektromagnetischer Strahlung im ultravioletten (UV-Licht) und/oder sichtbaren Spektralbereich durchgeführt wird.

2. Verfahren nach Anspruch 1, **dadurch gekennzeichnet, dass** die zugesetzte Menge des zumindest einen Fotoinitiators in der Vorvernetzung maximal gleich groß, bevorzugt kleiner, ist, als die Menge des zumindest einen Fotoinitiators, die für die Nachvernetzung verwendet wird.

3. Verfahren nach Anspruch 2, **dadurch gekennzeichnet, dass** der Anteil des zumindest einen Fotoinitiators an der

Polymerdispersion für die Vorvernetzung zwischen 0,2 phr und 5,0 phr beträgt.

4. Verfahren nach Anspruch 2 oder 3, **dadurch gekennzeichnet, dass** der Anteil des zumindest einen Fotoinitiators an der Polymerdispersion für die Nachvernetzung zwischen 0,5 phr und 5,0 phr beträgt.

5. Verfahren nach einem der Ansprüche 1 bis 4, **dadurch gekennzeichnet, dass** die Polymerdispersion in der Vorvernetzung zumindest zweimal bestrahlt wird.

6. Verfahren nach einem der Ansprüche 1 bis 5, **dadurch gekennzeichnet, dass** zumindest ein Fotoinitiator verwendet wird, der ausgewählt ist aus einer Gruppe umfassend 2-Hydroxy-2-methyl-1-phenylpropanon, Phenylglyoxalsäuremethylester, 2,4,6-Trimethylbenzoylphenylphosphinsäure Ethylester, Methylbenzoylformiat, 1-[4-(2-Hydroxyethoxy)-phenyl]-2-hydroxy-methyl-1-propanon-1-on, 2-Dimethylamino-2-(4-methylbenzyl)-1-(4-morpholin-4-ylphenyl)-butan-1-on, 2 Methyl-1-[4-(methylthio)phenyl]-2-morpholinopropan-1-on, 2,4,6-Trimethylbenzoyldiphenylphosphinoxid.

7. Verfahren nach einem der Ansprüche 1 bis 6, **dadurch gekennzeichnet, dass** die Vorvernetzung an einem Film durchgeführt wird, insbesondere mit einer Schichtdicke von maximal 2 mm.

8. Verfahren nach einem der Ansprüche 1 bis 7, **dadurch gekennzeichnet, dass** für die Nachvernetzung zumindest zwei Strahlungsquellen verwendet werden.

9. Verfahren nach einem der Ansprüche 1 bis 8, **dadurch gekennzeichnet, dass** die Nachvernetzung mit einer höheren Strahlungsdosis durchgeführt wird, als die Vorvernetzung.

10. Verfahren nach Anspruch 9, **dadurch gekennzeichnet, dass** die Nachvernetzung mit einer Strahlungsdosis durchgeführt wird, die zwischen 150 % und 500 % der Strahlungsdosis für die Vorvernetzung beträgt.

11. Verfahren nach einem der Ansprüche 1 bis 10, **dadurch gekennzeichnet, dass** der Vorvernetzung und/oder der Nachvernetzung zumindest ein Covernetzer mit zumindest einer Thiolgruppe zugesetzt wird.

12. Verfahren nach Anspruch 11, **dadurch gekennzeichnet, dass** die zugesetzte Menge des zumindest einen Covernetzers in der Vorvernetzung maximal gleich groß, bevorzugt kleiner, ist, als die Menge des zumindest einen Covernetzers, die für die Nachvernetzung verwendet wird.

13. Verfahren nach Anspruch 12, **dadurch gekennzeichnet, dass** der Anteil des zumindest einen Covernetzers an der Polymerdispersion für die Vorvernetzung zwischen 0,5 phr und 2,0 phr beträgt.

14. Verfahren nach Anspruch 12 oder 13, **dadurch gekennzeichnet, dass** der Anteil des zumindest einen Covernetzers an der Polymerdispersion für die Nachvernetzung zwischen 0,5 phr und 2,5 phr beträgt.

15. Verfahren nach einem der Ansprüche 11 bis 14, **dadurch gekennzeichnet, dass** als Covernetzer Trimethylolpropan-tris-3-mercaptopropionat oder Pentaerythritol tetrakis-3-mercaptopropionat verwendet wird.

16. Verfahren nach einem der Ansprüche 1 bis 15, **dadurch gekennzeichnet, dass** der vorvernetzten Polymerdispersion vor der Nachvernetzung zumindest ein Alterungsschutzmittel zugesetzt wird.

17. Verfahren nach Anspruch 16, **dadurch gekennzeichnet, dass** als Alterungsschutzmittel Vitamin E und/oder ein sterisch gehindertes Phenol verwendet wird.

18. Verfahren nach einem der Ansprüche 1 bis 17, **dadurch gekennzeichnet, dass** zwischen der Vorvernetzung und der Nachvernetzung eine Trocknung des vorvernetzten Elastomers bis zu einer Restfeuchte von maximal 6 % durchgeführt wird.

19. Verfahren nach einem der Ansprüche 1 bis 18, **dadurch gekennzeichnet, dass** der Polymerdispersion vor der Vorvernetzung zumindest ein Tensid mit zumindest einem photochemisch aktiven Zentrum, insbesondere mit einer Doppelbindung, zugesetzt wird.

20. Verfahren nach einem der Ansprüche 1 bis 19, **dadurch gekennzeichnet, dass** für die Bestrahlung eine mit Gallium

dotierte Quecksilber-Hochdruckdampflampe verwendet wird.

21. Verfahren nach einem der Ansprüche 1 bis 20, **dadurch gekennzeichnet, dass** die Nachvernetzung unter Inertgasatmosphäre durchgeführt wird.

22. Verfahren zur Herstellung eines Tauchartikels aus zumindest einem Latex bei dem eine Form mit einer äußeren Kontur, die dem herzustellenden Tauchartikel entspricht, für eine vorbestimmbare Zeit in ein Tauchbad, enthaltend den zumindest einen Latex, getaucht wird und danach der Tauchartikel verfestigt und/oder getrocknet wird, **dadurch gekennzeichnet, dass** der Latex nach einem Verfahren entsprechend einem der Ansprüche 1 bis 21 vernetzt wird.

23. Verfahren nach Anspruch 22, **dadurch gekennzeichnet, dass** die Formgebung des Latex durch Tauchen der Form in das Tauchbad zwischen der Vorvernetzung und der Nachvernetzung des Latex durchgeführt wird.

24. Verfahren nach Anspruch 23, **dadurch gekennzeichnet, dass** die Nachvernetzung auf der Form durchgeführt wird.

25. Vorrichtung zur Herstellung eines Tauchartikels aus einem Latex, umfassend einen Reaktor (19), zumindest eine Tauchform (30) und zumindest ein Tauchbad, wobei dem Reaktor (19) zumindest eine erste Strahlungsquelle (22) zur Abgabe von elektromagnetischer Strahlung im ultravioletten (UV-Licht) und/oder sichtbaren Spektralbereich zugeordnet ist, **dadurch gekennzeichnet, dass** nach dem zumindest einem Tauchbad eine weitere Strahlungsquelle zur Abgabe von elektromagnetischer Strahlung im ultravioletten (UV-Licht) und/oder sichtbaren Spektralbereich angeordnet ist.

26. Vorrichtung nach Anspruch 25, **dadurch gekennzeichnet, dass** nach dem zumindest einen Tauchbad eine Rollliereinheit angeordnet ist, mit der die Tauchartikel zumindest teilweise gerollt werden können, und die weitere Strahlungsquelle der Rollliereinheit zugeordnet ist.

27. Vorrichtung nach einem der Ansprüche 25 oder 26, **dadurch gekennzeichnet, dass** zumindest zwei weitere Strahlungsquellen in Förderrichtung der Tauchartikel hintereinander angeordnet sind.

28. Vorrichtung nach einem der Ansprüche 25 bis 27, **dadurch gekennzeichnet, dass** die erste Strahlungsquelle (19) und/oder die weitere Strahlungsquelle eine mit Gallium dotierte Quecksilber-Hochdruckdampflampe ist.

29. Vorrichtung nach einem der Ansprüche 25 bis 28, **dadurch gekennzeichnet, dass** zumindest zwei Reaktoren (19, 20) mit jeweils zumindest einer ersten Strahlungsquelle (22, 23) in Produktionsrichtung hintereinander angeordnet sind.

30. Vorrichtung nach Anspruch 29, **dadurch gekennzeichnet, dass** zwischen den beiden Reaktoren (19, 20) zumindest ein Behälter (24) angeordnet ist, der gegebenenfalls ein Rührwerk aufweist.

31. Handschuh aus einem vernetzten Elastomer, **dadurch gekennzeichnet, dass** dieser nach einem Verfahren nach einem der Ansprüche 1 bis 24 hergestellt ist und eine Reißfestigkeit von zumindest 14 $N/mm^2$ aufweist.

## Claims

1. Method for producing a crosslinked elastomer by radiating a polymer dispersion of at least one crosslinkable polymer with electromagnetic radiation in the ultraviolet (UV light) and/or visible spectral range, and the crosslinking is performed in at least two stages as pre-crosslinking and post-crosslinking, and at least one photoiniatiator is added to the polymer dispersion prior to the pre-crosslinking to trigger the crosslinking reaction, **characterized in that** a photoinitator is added once again to the pre-crosslinked polymer dispersion prior to and/or during the post-crosslinking and the post-crosslinking is also performed with electromagnetic radiation in the ultraviolet (UV light) and/or visible spectral range.

2. Method according to claim 1, **characterized in that** the added amount of the at least one photoinitiator in the pre-crosslinking is at a maximum the same, preferably lower, than the amount of the at least one photoinitiator used for the post-crosslinking.

3. Method according to claim 2, **characterized in that** the proportion of the at least one photoinitiator in the polymer

dispersion for the pre-crosslinking is between 0.2 phr and 5.0 phr.

4. Method according to claim 2 or 3, **characterized in that** the proportion of the at least one photoinitiator in the polymer dispersion for the post-crosslinking is between 0.5 phr and 5.0 phr.

5. Method according to one of claims 1 to 4, **characterized in that** the polymer dispersion is radiated at least twice in the pre-crosslinking.

6. Method according to one of claims 1 to 5, **characterized in that** at least one photoinitiator is used, which is selected from a group comprising 2-hydroxy-2-methyl-1-phenylpropanone, phenylglyoxylic acid methyl ester, 2,4,6-trimethylbenzoylphenylphosphinic acid ethyl ester, methylbenzoylformiate, 1-[4-(2-hydroxyethoxy)-phenyl]-2-hydroxy-methyl-1-propanone-1-one, 2-dimethylamino-2-(4-methylbenzyl)-1-(4-morpholine-4-ylphenyl)-butane-1-one, 2 methyl-1-[4-(methylthio)phenyl]-2-morpholinopropane-1-one, 2,4,6-trimethylbenzoyl diphenylphosphine oxide.

7. Method according to one of claims 1 to 6, **characterized in that** the pre-crosslinking is performed on a film, in particular with a layer thickness of a maximum of 2 mm.

8. Method according to one of claims 1 to 7, **characterized in that** for the post-crosslinking at least two radiation sources are used.

9. Method according to one of claims 1 to 8, **characterized in that** the post-crosslinking is performed with a higher radiation dose than the pre-crosslinking.

10. Method according to claim 9, **characterized in that** the post-crosslinking is performed with a radiation dose, which is between 150 % and 500 % of the radiation dose for the pre-crosslinking.

11. Method according to one of claims 1 to 10, **characterized in that** at least one co-crosslinking agent with at least one thiol group is added to the pre-crosslinking and/or the post-crosslinking.

12. Method according to claim 11, **characterized in that** the added amount of the at least one co-crosslinking agent in the pre-crosslinking is at a maximum the same, preferably lower, than the amount of the at least one co-crosslinking agent used for the post-crosslinking.

13. Method according to claim 12, **characterized in that** the proportion of the at least one co-crosslinking agent in the polymer dispersion for the pre-crosslinking is between 0.5 phr and 2.0 phr.

14. Method according to claim 12 or 13, **characterized in that** the proportion of the at least one co-crosslinking agent in the polymer dispersion for the post-crosslinking is between 0.5 phr and 2,5 phr.

15. Method according to one of claims 11 to 14, **characterized in that** trimethylolpropane tris-3-mercaptopropionate or pentaerythritol tetrakis-3-mercaptopropionate is used as a co-crosslinking agent.

16. Method according to one of claims 1 to 15, **characterized in that** at least one ageing protection means is added to the pre-crosslinked polymer dispersion prior to the post-crosslinking.

17. Method according to claim 16, **characterized in that** as the ageing protection means vitamin E and/or a sterically hindered phenol is used.

18. Method according to one of claims 1 to 17, **characterized in that** between the pre-crosslinking and the post-crosslinking a drying of the pre-crosslinked elastomer is performed up to a residual moisture content of a maximum of 6 %.

19. Method according to one of claims 1 to 18, **characterized in that** at least one surfactant with at least one photochemically active center, in particular with a double bond, is added to the polymer dispersion prior to the pre-crosslinking.

20. Method according to one of claims 1 to 19, **characterized in that** for the radiation a mercury high pressure vapor lamp doped with gallium is used.

**21.** Method according to one of claims 1 to 20, **characterized in that** the post-crosslinking is performed in an inert gas atmosphere.

**22.** Method for producing an immersion article from at least one latex in which a mold with an external contour, which corresponds to that of the immersion article to be produced, is immersed for a prespecificable period in an immersion bath containing the at least one latex, and afterwards the immersion article is hardened and/or dried, **characterized in that** the latex is crosslinked according to a method as claimed in one of claims 1 to 21.

**23.** Method according to claim 22, **characterized in that** the shaping of the latex by immersing the mold into the immersion bath is performed between the pre-crosslinking and the post-crosslinking of the latex.

**24.** Method according to claim 23, **characterized in that** the post-crosslinking is performed on the mold.

**25.** Device for producing an immersion article from latex, comprising a reactor (19), at least one immersion mold (30) and at least one immersion bath, in which at least one radiation source (22) is assigned to the reactor (19) for the emission of electromagnetic radiation in the ultraviolet (UV light) and/or visible spectral range, **characterized in that** after the at least one immersion bath an additional radiation source is arranged for the emission of electromagnetic radiation in the ultraviolet (UV light) and/or visible spectral range.

**26.** Device according to claim 25, **characterized in that** after the at least one immersion bath a rolling unit is arranged, by means of which the immersion articles can be rolled at least partly, and the additional radiation source is assigned to the rolling unit.

**27.** Device according to one of claims 25 or 26, **characterized in that** the at least two additional radiation sources are arranged behind one another in the conveying direction of the immersion articles.

**28.** Device according to one of claims 25 to 27, **characterized in that** the first radiation source (19) and/or the additional radiation source is a mercury high pressure vapor lamp doped with gallium.

**29.** Device according to one of claims 25 to 28, **characterized in that** at least two reactors (19, 20) each with at least one first radiation source (22, 23) are arranged behind one another in production direction.

**30.** Device according to claim 29, **characterized in that** between the two reactors (19, 20) at least one container (24) is arranged, which optionally comprises an agitator.

**31.** Glove made from a crosslinked elastomer, **characterized in that** the latter is produced by a method according to one of claims 1 to 24 and has a tearing resistance of at least 14 $N/mm^2$.

## Revendications

**1.** Procédé de fabrication d'un élastomère réticulé par irradiation d'une dispersion de polymères, constituée d'au moins un polymère réticulable, avec un rayonnement électromagnétique dans le domaine spectral ultraviolet (lumière UV) et/ou visible, la réticulation étant mise en oeuvre en au moins deux étapes, sous forme d'une pré réticulation et d'une post-réticulation, et la dispersion de polymères étant additionnée d'au moins un photo-amorceur pour déclencher la réaction de réticulation avant la pré-réticulation, **caractérisé en ce que** la dispersion de polymères pré-réticulée est encore une fois additionnée d'au moins un photo-amorceur avant et/ou pendant la post-réticulation, et la post-réticulation est elle aussi mise en oeuvre avec un rayonnement électromagnétique dans le domaine spectral ultraviolet (lumière UV) et/ou visible.

**2.** Procédé selon la revendication 1, **caractérisé en ce que** la quantité ajoutée du ou des photo-amorceurs au cours de la pré-réticulation est au maximum égale, de préférence inférieure, à la quantité du ou des photo-amorceurs qui sont utilisés pour la post-réticulation.

**3.** Procédé selon la revendication 2, **caractérisé en ce que** la proportion du ou des photo-amorceurs par rapport à la dispersion de polymères est pour la pré-réticulation comprise entre 0,2 phr et 5,0 phr.

**4.** Procédé selon la revendication 2 ou 3, **caractérisé en ce que** la proportion du ou des photo-amorceurs par rapport

à la dispersion de polymères est, pour la post-réticulation, comprise entre 0,5 phr et 5,0 phr.

5. Procédé selon l'une des revendications 1 à 4, **caractérisé en ce que** la dispersion de polymères est, au cours de la pré-réticulation, irradiée au moins deux fois.

6. Procédé selon l'une des revendications 1 à 5, **caractérisé en ce qu'**on utilise au moins un photo-amorceur qui est choisi dans le groupe consistant en la 2-hydroxy-2-méthyl-1-phénylpropanone, l'ester méthylique de l'acide phénylglyoxalique, l'ester éthylique de l'acide 2,4,6-triméthylbenzoylphénylphosphinique, le benzoylformiate de méthyle, la 1-[4-(2-hydroxyéthoxy)-phényl]-2-hydroxy-méthyl-1-propanon-1-one, la 2-diméthylamino-2-(4-méthylbenzyl)-1-(4-morpholin-4-ylphényl)-butan-1-one, la 2-méthyl-1-[4-(méthylthio)phényl]-2-morpholinopropan-1-one, l'oxyde de 2,4,6-triméthylbenzoyldiphénylphosphine.

7. Procédé selon l'une des revendications 1 à 6, **caractérisé en ce que** la pré-réticulation est mise en oeuvre sur un film, ayant en particulier une épaisseur de feuil au maximum de 2 mm.

8. Procédé selon l'une des revendications 1 à 7, **caractérisé en ce qu'**on utilise pour la post-réticulation au moins deux sources de rayonnement.

9. Procédé selon l'une des revendications 1 à 8, **caractérisé en ce que** la post-réticulation est mise en oeuvre avec une dose de rayonnement plus élevée que la pré-réticulation.

10. Procédé selon la revendication 9, **caractérisé en ce que** la post-réticulation est mise en oeuvre avec une dose de rayonnement qui est comprise entre 150 % et 500 % de la dose de rayonnement pour la pré-réticulation.

11. Procédé selon l'une des revendications 1 à 10, **caractérisé en ce qu'**on ajoute au moins un co-réticulant ayant au moins un groupe thiol à la pré-réticulation et/ou à la post-réticulation.

12. Procédé selon la revendication 11, **caractérisé en ce que** la quantité ajoutée du ou des co-réticulants est lors de la pré-réticulation au maximum égale, de préférence inférieure, à la quantité du ou des co-réticulants qui sont utilisés pour la post-réticulation.

13. Procédé selon la revendication 12, **caractérisé en ce que** la proportion du ou des co-réticulants par rapport à la dispersion de polymères est pour la pré-réticulation comprise entre 0,5 phr et 2,0 phr.

14. Procédé selon la revendication 12 ou 13, **caractérisé en ce que** la proportion du ou des co-réticulants par rapport à la dispersion de polymères est, pour la post-réticulation, comprise entre 0,5 phr et 2,5 phr.

15. Procédé selon l'une des revendications 11 à 14, **caractérisé en ce qu'**on utilise en tant que co-réticulant le tris-3-mercaptopropionate de triméthylolpropane ou le tétrakis-3-mercaptopropionate de pentaérythritol.

16. Procédé selon l'une des revendications 1 à 15, **caractérisé en ce qu'**au moins un agent de protection contre le vieillissement est ajouté à la dispersion de polymères pré-réticulée avant la post-réticulation.

17. Procédé selon la revendication 16, **caractérisé en ce qu'**on utilise en tant qu'agent de protection contre le vieillissement la vitamine E et/ou un phénol à empêchement stérique.

18. Procédé selon l'une des revendications 1 à 17, **caractérisé en ce qu'**on procède, entre la pré-réticulation et la post-réticulation, à un séchage de l'élastomère pré-réticulé, jusqu'à une teneur résiduelle en humidité au maximum de 6 %.

19. Procédé selon l'une des revendications 1 à 18, **caractérisé en ce qu'**on ajoute à la dispersion de polymères avant la pré-réticulation au moins un tensioactif ayant au moins un centre photochimiquement actif, comportant de préférence une double liaison.

20. Procédé selon l'une des revendications 1 à 19, **caractérisé en ce qu'**on utilise pour l'irradiation une lampe à vapeur de mercure haute pression dopée au gallium.

21. Procédé selon l'une des revendications 1 à 20, **caractérisé en ce que** la post-réticulation est mise en oeuvre sous une atmosphère d'un gaz inerte.

**22.** Procédé de fabrication d'un article au trempé, constitué d'au moins un latex, dans lequel un moule ayant un contour extérieur correspondant à l'article au trempé à fabriquer est pendant un laps de temps pouvant être prédéfini immergé dans un bain de trempage, contenant le ou les latex, puis l'article au trempé est solidifié et/ou séché, **caractérisé en ce que** le latex est réticulé par un procédé correspondant à l'une des revendications 1 à 21.

**23.** Procédé selon la revendication 22, **caractérisé en ce que** le moulage du latex est mis en oeuvre par immersion du moule dans le bain de trempage entre la pré-réticulation et la post-réticulation du latex.

**24.** Procédé selon la revendication 23, **caractérisé en ce que** la post-réticulation est mise en oeuvre sur le moule.

**25.** Dispositif pour la fabrication d'un article au trempé constitué d'un latex, comprenant un réacteur (19), au moins un moule de trempage (30) et au moins un bain de trempage, au moins une source de rayonnement (22), destinée à émettre un rayonnement électromagnétique dans le domaine spectral ultraviolet (lumière UV) et/ou visible, étant affectée au réacteur (19), **caractérisé en ce que**, après le ou les bains de trempage, une source de rayonnement supplémentaire est disposée, pour émettre un rayonnement électromagnétique dans le domaine spectral ultraviolet (lumière UV) et/ou visible.

**26.** Dispositif selon la revendication 25, **caractérisé en ce qu'**une unité de calandrage est disposée après le ou les bains de trempage, à l'aide de laquelle il est possible de calandrer au moins partiellement les articles au trempé, et que la source de rayonnement supplémentaire est affectée à l'unité de calandrage.

**27.** Dispositif selon l'une des revendications 25 ou 26, **caractérisé en ce qu'**au moins deux sources de rayonnement supplémentaires sont disposées l'une derrière l'autre dans la direction du transport des articles au trempé.

**28.** Dispositif selon l'une des revendications 25 à 27, **caractérisé en ce que** la première source de rayonnement (19) et/ou la source de rayonnement supplémentaire sont des lampes à vapeur de mercure haute pression dopées au gallium.

**29.** Dispositif selon l'une des revendications 25 à 28, **caractérisé en ce qu'**au moins deux réacteurs (19, 20) sont disposés l'un derrière l'autre dans la direction de la production, chacun ayant au moins une première source de rayonnement (22, 23).

**30.** Dispositif selon la revendication 29, **caractérisé en ce qu'**au moins un récipient (24), qui éventuellement comprend un agitateur, est disposé entre les deux réacteurs (19, 20).

**31.** Gant en un élastomère réticulé, **caractérisé en ce qu'**il est fabriqué par un procédé selon l'une des revendications 1 à 24, et présente une résistance à la déchirure d'au moins 14 $N/mm^2$.

**Fig.1**

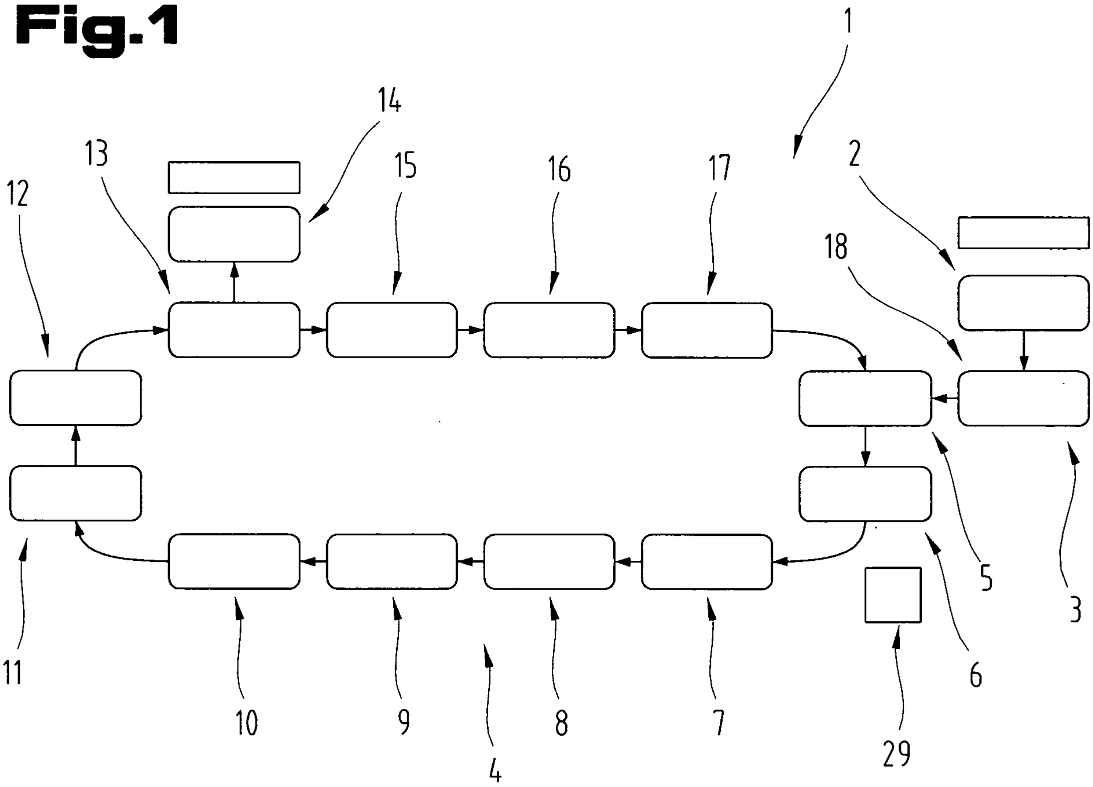
1
2
3
4
5
6
7
8
9
10
11
12
13
14
15
16
17
18
29

**Fig.2**

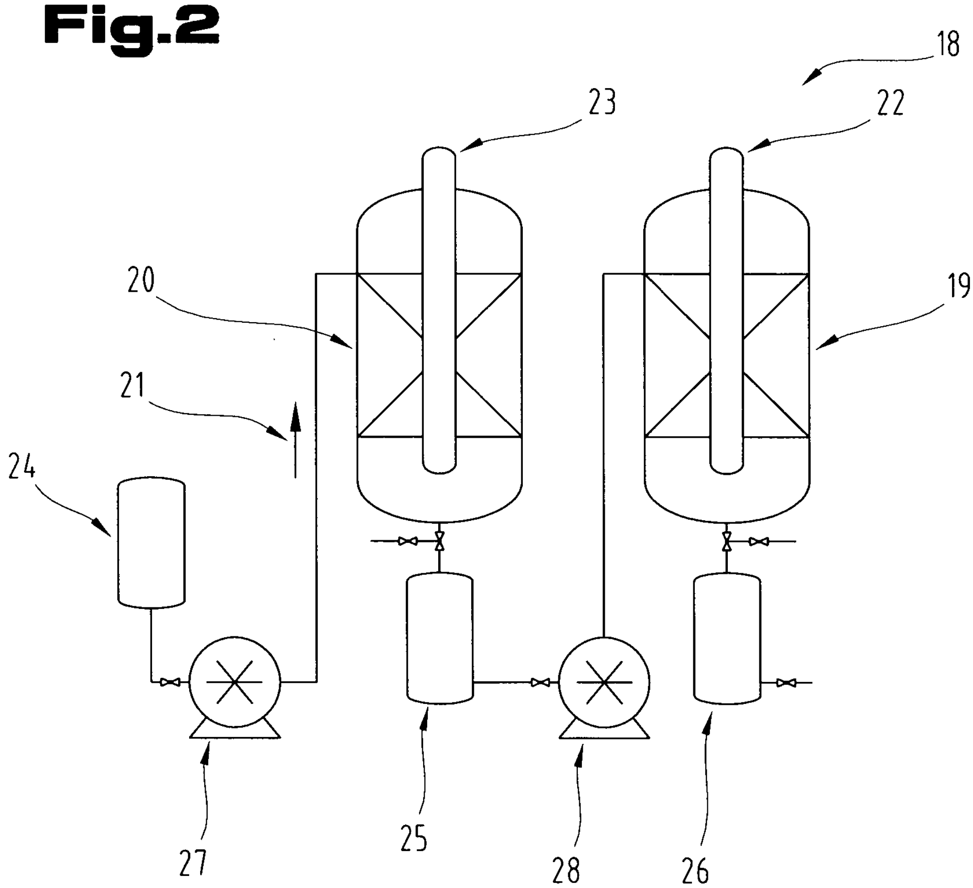
18
19
20
21
22
23
24
25
26
27
28

Fig.3

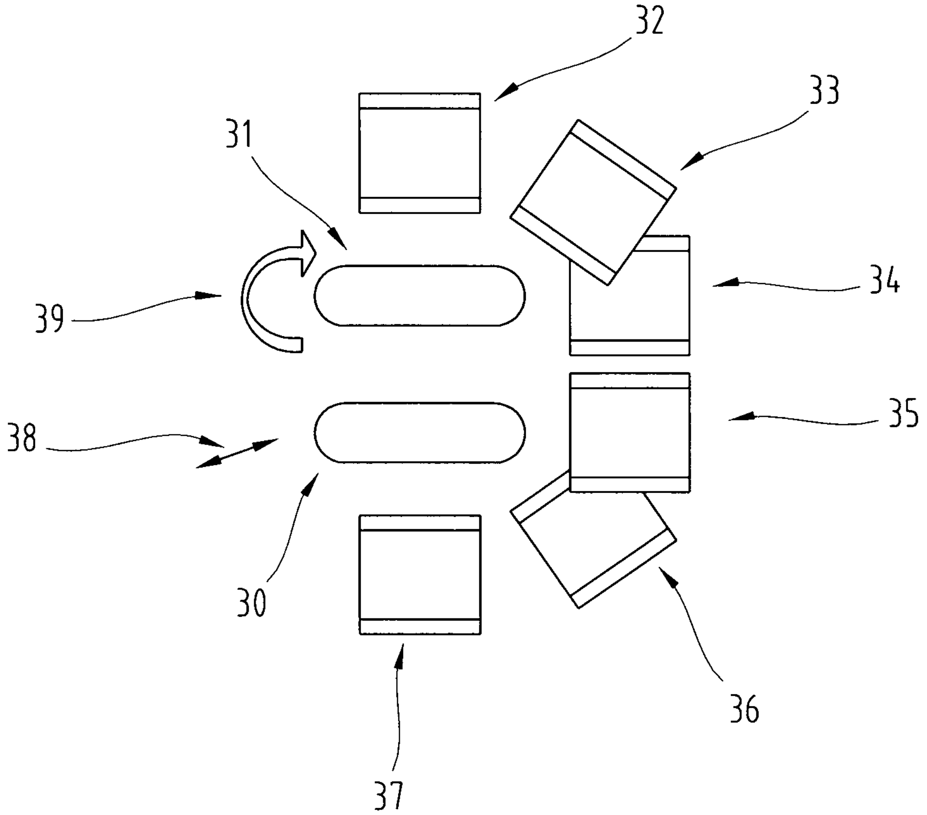


Fig.4 Fig.5 Fig.6

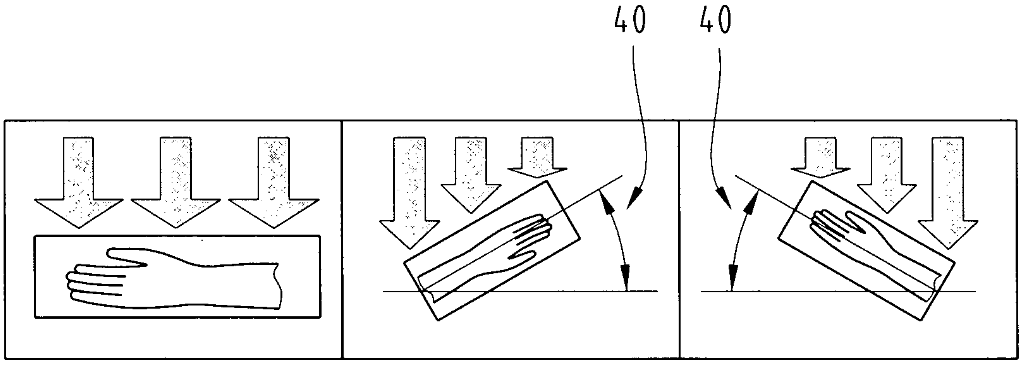

**Fig.7**

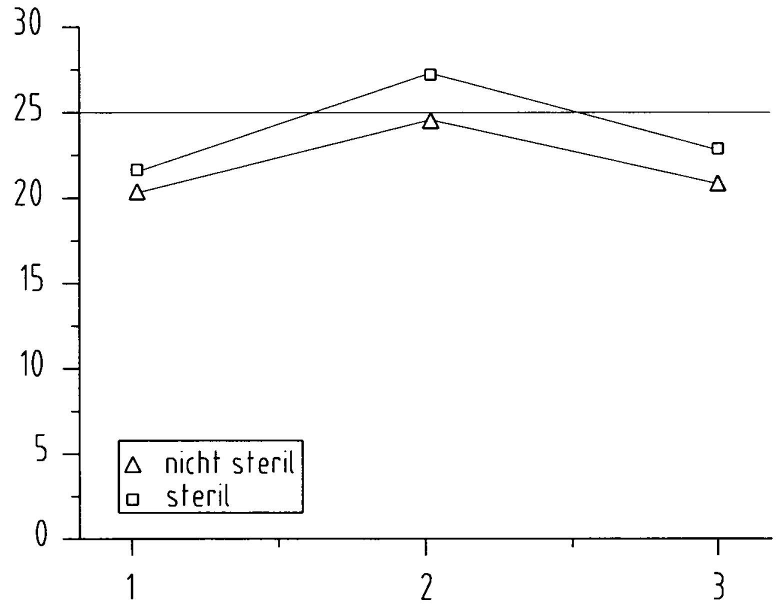
30
25
20
15
10
5
0
1
2
3
nicht steril
steril

**Fig.8**

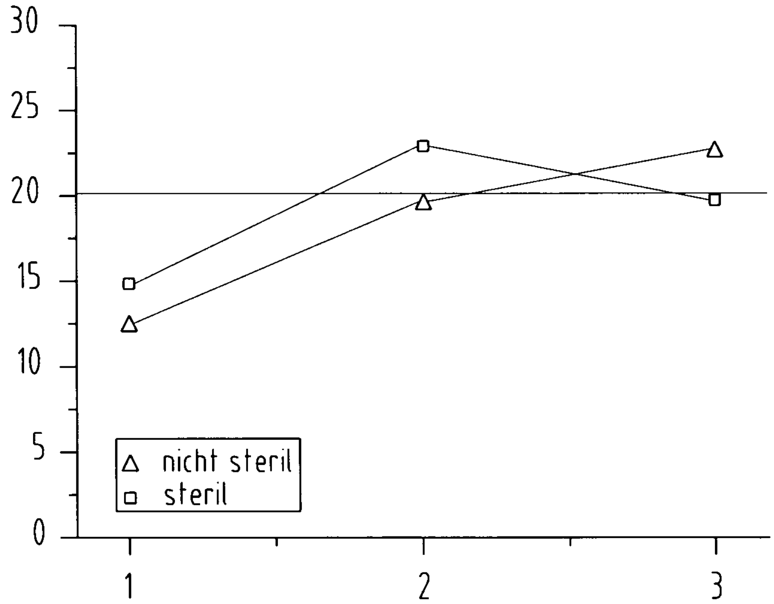
30
25
20
15
10
5
0
1
2
3
nicht steril
steril

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1762586 A2
- EP 0856294 A
- EP 0856294 A1


### In der Beschreibung aufgeführte Nicht-Patentliteratur


- Veröffentlichung. Röthemeyer/Sommer. Carl Hanser Verlag, 2001
- Annu. Book ASTM Stand. 09.01. 2002